(19) Europäisches Patentamt
European Patent Office
Office européen des brevets

(11) Veröffentlichungsnummer: **0 156 347 B1**

# (12) EUROPÄISCHE PATENTSCHRIFT

(45) Veröffentlichungstag der Patentschrift: **04.09.91**

(51) Int. Cl.$^5$: **C07H 17/00**, C12Q 1/34, //C07D265/38,C07D265/36

(21) Anmeldenummer: **85103496.7**

(22) Anmeldetag: **25.03.85**

Die Akte enthält technische Angaben, die nach dem Eingang der Anmeldung eingereicht wurden und die nicht in dieser Patentschrift enthalten sind.

## (54) Glycoside von Resorufin-Derivaten, Verfahren zu deren Herstellung sowie deren Verwendung zur Bestimmung der Aktivität von Glycosidasen.

(30) Priorität: **29.03.84 DE 3411574**

(43) Veröffentlichungstag der Anmeldung: **02.10.85 Patentblatt 85/40**

(45) Bekanntmachung des Hinweises auf die Patenterteilung: **04.09.91 Patentblatt 91/36**

(84) Benannte Vertragsstaaten: **AT BE CH DE FR GB IT LI LU NL SE**

(56) Entgegenhaltungen:
**EP-A- 0 074 761**
**DE-A- 2 752 501**
**US-A- 3 378 463**
**US-A- 3 391 104**
**US-A- 3 950 322**

**CHEMICAL ABSTRACTS, Band 77, Nr. 15, 9. Oktober 1972, Seite 420, Zusammenfassung Nr. 101493h, Columbus, Ohio, US; E. RUZIKKA et al.: "Reactions of nitrosophenols. VI. Preparation of some hydroxyphenoxazones", & COLLECT. CZECH. CHEM. COMMUN. 1972, 37(6), 1905-7**

**ANALYTICA CHIMICA ACTA, Band 163, 1984, Seiten 67-72, Elsevier Science Publishers B.V., Amsterdam, NL; J. HOFMANN et al.: "Immobilized enzyme kinetics analyzed by flow-through microfluorimetry"**

(73) Patentinhaber: **BOEHRINGER MANNHEIM GMBH**
**Sandhofer Strasse 116**
**W-6800 Mannheim 31(DE)**

(72) Erfinder: **Klein, Christian, Dr.**
**Johann-Baur-Strasse 40**
**W-8120 Weilheim(DE)**
Erfinder: **Batz, Hans-Georg, Dr. rer. nat.**
**Traubinger Strasse 63**
**W-8132 Tutzing(DE)**
Erfinder: **Hofmann, Jürgen, Dr. rer. nat.**
**Im Gefälle 42**
**W-3550 Marburg(DE)**
Erfinder: **Sernetz, Manfred, Prof. Dr. med. vet.**
**Amselweg 15**
**W-6301 Wettenberg 1(DE)**

EP 0 156 347 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Glycosidasen erfüllen im menschlichen und tierischen Organismus eine vielfache physiologische Funktion. So spielt beispielsweise die β-D-Galactosidase eine gewichtige Rolle im Kohlenhydratstoffwechsel, da durch sie die Hydrolyse der Lactose erfolgt. Darüberhinaus stellt die β-D-Galactosidase das Schlüsselenzym beim Abbau von Glycolipiden, Mucopolysacchariden und Glycoproteinen dar. Als weitere physiologisch bedeutsame Glycosidasen sind zu nennen: Die α-D-Galactosidase, die α-D- und β-D-Glucosidase sowie α-D-Mannosidase.

Über ihren physiologischen Stellenwert hinaus haben die Glycosidasen in den letzten Jahren im diagnostischen sowie im biotechnologischen Bereich an Bedeutung gewonnen. So werden beispielsweise diese Enzyme in zunehmendem Maße als Indikator-Enzym für Enzymimmunoassays eingesetzt. Besonders bevorzugt wird in diesem Zusammenhang die β-D-Galactosidase [siehe beispielsweise Annals of Clinical Biochemistry 16, 221 - 240 (1979)].

Die Bestimmung der Aktivität der Glycosidasen spielt demnach sowohl in der klinischen Chemie als auch in der Diagnostik eine zunehmende Rolle. Hierzu wird ganz allgemein die Glycosidase-haltige Probe mit einem geeigneten Substrat versetzt. Das Substrat wird von dem Enzym gespalten. Eines der Spaltprodukte wird in geeigneter Weise nachgewiesen. Es kann entweder das durch Einwirkung des Enzyms freigesetzte Glycon oder das Aglycon gemessen werden. In der Regel wird letzteres bestimmt.

Als Substrat eignen sich oft die natürlichen Substrate der nachzuweisenden Enzyme. Besonders bevorzugt werden jedoch Glycoside verwendet, bei denen das Aglycon einen spektroskopisch leicht nachweisbaren Rest darstellt.

Es sind eine Reihe von Glycosidase-Substraten bekannt, bei denen nach der Spaltung durch die Glycosidase das Aglycon spektroskopisch im sichtbaren oder auch UV-Bereich sowie fluorometrisch gemessen werden kann.

So sind in Biochem. Z. 333, 209 (1960) Phenyl-β-D-galactosid sowie einige weitere am aromatischen Ring substituierte Derivate (z. B. o-Nitrophenyl- und p-Nitrophenyl-β-D-galactosid) als Substrate der β-D-Galactosidase beschrieben. Die durch Hydrolyse freigesetzten Phenole werden photometrisch im UV-Bereich bzw. bei den Nitrophenolen im kurzwelligen sichtbaren Wellenlängenbereich bestimmt. Auch kann als Indikatorreaktion eine oxidative Kupplung mit Aminoantipyrin angeschlossen werden [Analytical Biochem. 40, 281 (1971)].

Für histochemische Untersuchungen werden Naphthyl-β-D-galactoside verwendet, so z. B. die 1-Naphthyl-Verbindung in Histochemie 35, 199 (1973), das 6-Brom-2-naphthyl-Derivat in J. Biol. Chem. 195, 239 (1952) oder das Naphthol-β-D-galactosid [Histochemie 37, 89 (1973)]. Zur Visualisierung werden dabei die entstehenden Naphthole mit verschiedenen Diazoniumsalzen zu Azo-Farbstoffen umgesetzt.

Die Bestimmung von Enzymaktivitäten mit fluorogenen Substraten ist weitverbreitet, da gegenüber den photometrischen Methoden die Empfindlichkeit fluorometrischer Bestimmungen oft um einige Zehnerpotenzen erhöht ist. In manchen Fällen muß mit fluorogenen Substraten gearbeitet werden, beispielsweise bei der Untersuchung enzymatischer Aktivität in Zellen mit automatischen Geräten zur Zelldifferenzierung (Zytofluorometrie) sowie bei der Analyse immobilisierter Enzyme mit Durchflußmikrofluorometrie. In anderen Fällen, beispielsweise bei der Bestimmung enzymatischer Markierung von Testsystemen (Enzymimmunoassays), wird der Multiplikationseffekt der enzymatischen Katalyse durch Verwendung fluorogener Substrate beträchtlich verstärkt.

Aus der US-Patentschrift Nr. 3378463 sind verschiedene Acylresorufine als fluorogene Substrate zur Bestimmung der Aktivität bestimmter Enzyme bekannt.

Die bisher bekannten fluorogenen Substrate für β-D-Galactosidase und andere Glycosidasen besitzen als Fluorophore Derivate von Fluorescein, Indoxyl oder Methylumbelliferon. Für die kinetische Analyse komplexer Systeme besitzen diese Verbindungen jedoch schwerwiegende Nachteile. Die disubstituierten Derivate von Fluorescein werden in einer mehrstufigen Reaktionsfolge hydrolysiert. Monosubstituierte Fluorescein-Glycoside fluoreszieren bereits selbst. Indoxyl-Derivate unterliegen nach ihrer enzymatischen Spaltung einer Reihe von chemischen Umwandlungen, die ebenfalls die kinetische Analyse komplizieren. Derivate von Methylumbelliferon müssen im UV angeregt werden. Hierbei kann die Eigenfluoreszenz von biologischen oder synthetischen Materialien stören. Darüberhinaus ist die UV-Anregung, insbesondere bei Laseroptik, kostenaufwendig. Die meisten fluorogenen Substrate führen zu Reaktionsprodukten, die nur eine geringe Löslichkeit aufweisen, so daß sie für die kinetische Analyse von Enzymaktivitäten, wofür gerade gute Löslichkeit von Substrat und Produkt erforderlich ist, nicht geeignet sind.

Es bestand daher weiterhin ein Bedarf an Substraten, mit denen verschiedene Glycosidasen auf einfache, schnelle und zuverlässige Weise bestimmt werden können und die möglicherweise sowohl bei photometrischen als auch fluorometrischen Bestimmungsverfahren eingesetzt werden können. Aufgabe der

vorliegenden Erfindung war es, diesen Bedarf zu befriedigen.

Gelöst wird diese Aufgabe durch die neuen Glycoside von Resorufin-Derivaten, die sich mit Hilfe von Glycosidasen in den Zuckeranteil und in die Resorufin-Derivate spalten lassen. Letzere sind gut wasserlösliche Verbindungen, die eine gut meßbare Absorption im sichtbaren Bereich aufweisen und sich ferner leicht zur Fluoreszenz anregen lassen.

Gegenstand der vorliegenden Erfindung sind demnach Glycoside von Resorufin-Derivaten der allgemeinen Formel Ia bzw. Ib

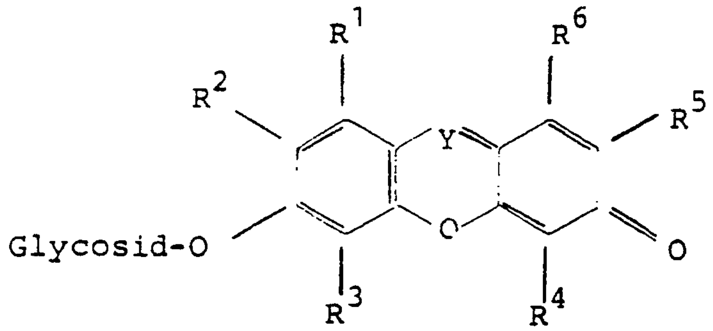

(I a)

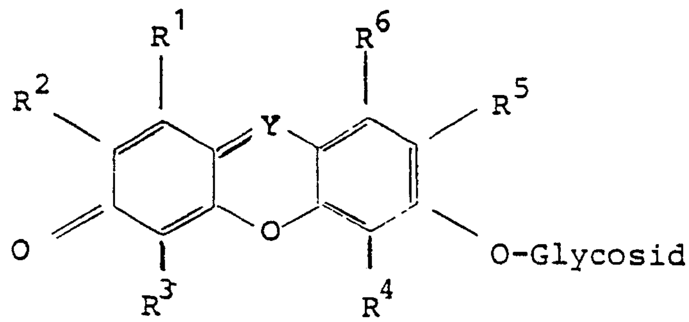

(I b)

in denen

$R^1$ Wasserstoff,

$R^2$, $R^3$ und $R^5$, die gleich oder verschieden sein können, Wasserstoff, Halogen oder eine Niederalkylgruppe,

$R^4$ und $R^6$, die gleich oder verschieden sein können, Wasserstoff, Halogen, eine Cyano-, Niederalkyl-, Niederalkoxy-, Carboxy-, Niederalkoxycarbonyl-, Carboxyniederalkyl-, Niederalkoxycarbonyl-niederalkyl- oder eine gegebenenfalls ein- oder zweifach substituierte Carboxamidogruppe oder die Gruppe

$-COO-(CH_2CH_2O)_nR^7$,

in der $R^7$ Wasserstoff oder eine Niederalkyl-Gruppe

und n eine ganze Zahl von 1 bis 4 bedeuten,

wobei $R^6$ zusätzlich eine Sulfonyl- oder Nitrogruppe vorstellen kann, und

Y ein Stickstoff oder die Gruppe N→O

bedeuten.

Die Glycoside von Resorufin-Derivaten der allgemeinen Formel I sind neue Verbindungen. Sie können nach an sich aus der Kohlenhydratchemie bekannten Methoden hergestellt werden.

Vorzugsweise werden in an sich bekannter Weise Resorufin-Derivate der allgemeinen tautomeren Formeln IIa und IIb

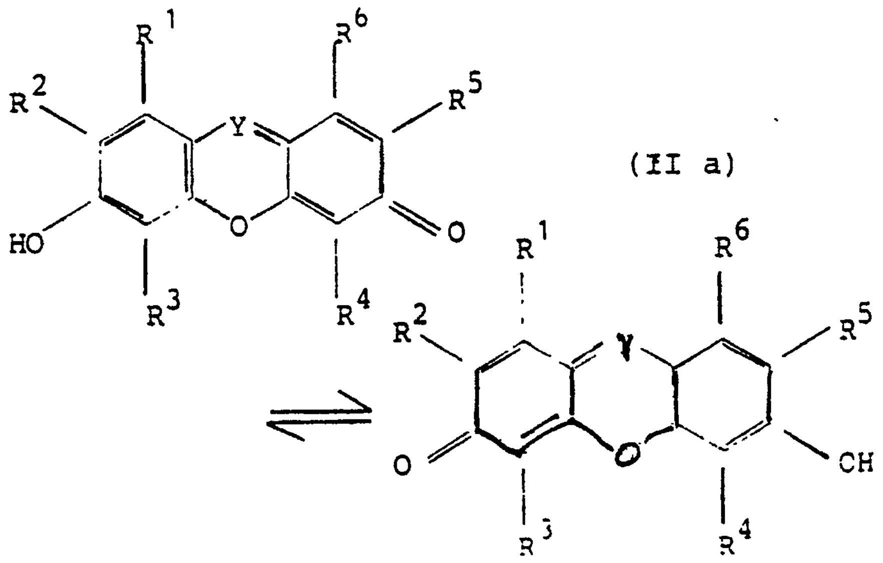

(II a)

(II b)

in denen
$R^1$ bis $R^6$ und Y die oben angegebene Bedeutung haben,
mit einem Mono- oder Oligo-Saccharid oder einem 1-Halogeno-Derivat hiervon, wobei jeweils alle Hydroxygruppen mit einer in der Kohlenhydratchemie üblichen Schutzgruppe substituiert sind, zu per-O-substituierten Glycosiden umgesetzt, aus denen durch Abspaltung der Schutzgruppen in an sich bekannter Weise die Glycoside von Resorufin-Derivaten der allgemeinen Formel I erhalten werden.

Die Umsetzung der Verbindungen der Formel II mit den per-O-substituierten 1-Halogeno-Sacchariden wird vorzugsweise in Gegenwart von Säurefängern, wie Alkalihydroxid oder -carbonat, in wäßrigem Aceton oder (unter Phasentransferbedingungen) in einem Wasser/Benzol- oder Wasser/Chloroform-Gemisch vorgenommen.

Darüberhinaus kann dieses Verfahren durchgeführt werden, indem man die Resorufin-Derivate der allgemeinen Formel II zunächst mittels Alkalihydroxid oder -alkoholat in die Alkalisalze bzw. mittels gegebenenfalls substituierter Amine in die Ammoniumsalze überführt und diese dann in dipolar aprotischen Lösungsmitteln wie Aceton, Dimethylsulfoxid, Dichlormethan, Tetrahydrofuran oder Dimethylformamid mit den per-O-substituierten 1-Halogeno-Sacchariden umsetzt.

Ferner haben sich bei der Synthese der per-O-substituierten Glycoside aus den Resorufin-Derivaten der allgemeinen Formel II und den per-O-substituierten 1-Halogeno-Sacchariden Zusätze von einzelnen Silbersalzen oder Gemischen von Silbersalzen (Silberoxid, -carbonat, -carbonat auf Celite, -triflat, -salicylat) und/oder von einzelnen Quecksilbersalzen oder Gemischen von Quecksilbersalzen (Quecksilberbromid, -cyanid, -acetat, -oxid), gegebenenfalls unter Verwendung von Trocknungsmitteln wie Calciumchlorid, Molekularsieb oder Drierit, in Lösungsmitteln, wie Methylenchlorid, Chloroform, Benzol, Toluol oder Dioxan, bewährt. Zur Synthese der α-verknüpften Glycoside wird zweckmäßigerweise eine Verbindung der allgemeinen Formel II mit einem Saccharid, dessen Hydroxygruppen mit einer Schutzgruppe, besonders bevorzugt mit einer Acetylgruppe, substituiert sind, in Gegenwart einer Lewissäure, wie z.B. Zinntetrachlorid, Aluminiumchlorid, vorzugsweise Zinkchlorid, geschmolzen (vgl. Chem. Ber. 66, 378-383 [1933] und Methods in Carbonydr. Chem. 2, 345-347 [1967]). Die Temperatur wird hierbei zwischen 80 und 150° C, bevorzugt zwischen 110 und 130° C gewählt.

Die so erhaltenen per-O-substituierten Glycoside von den Resorufin-Derivaten der allgemeinen Formel II sind ebenfalls neue Verbindungen.

Die Abspaltung der Schutzgruppen von den per-O-substituierten Glycosiden zu den Glycosiden der allgemeinen Formel I wird nach in der Kohlenhydratchemie gängigen Methoden [s. z. B. Advances Carbohydrate Chem. 12, 157 (1975)], beispielsweise bei den Acyl-Schutzgruppen mittels Natriummethylat oder Bariummethylat oder Ammoniak in Methanol, durchgeführt.

Unter Halogen in der Definition von $R^1$ bis $R^7$ ist Fluor, Chlor, Brom und Iod, vorzugsweise Chlor und Brom, zu verstehen.

Die "Niederalkylgruppe" in der Definition von $R^1$ bis $R^6$ enthält 1 bis 5, vorzugsweise 1 bis 3 Kohlenstoffatome, wobei die Methylgruppe besonders bevorzugt ist.

Die "Niederalkoxygruppe" in der Definition von $R^4$ und $R^6$ enthält 1 bis 5, vorzugsweise 1 bis 3 Kohlenstoffatome, wobei die Methoxygruppe besonders bevorzugt ist.

Die "Niederalkoxy-" bzw. die "Niederalkyl-"Reste der Niederalkoxycarbonyl-, Carboxy-niederalkyl-sowie der Niederalkoxycarbonyl-niederalkyl-Reste in der Definition der Substituenten $R^4$ und $R^6$ weisen ebenfalls 1 bis 5, vorzugsweise 1 bis 3 Kohlenstoffatome auf, wobei die Methoxy-Gruppe bzw. der Methylen-Rest besonders bevorzugt sind.

Als "in der Kohlenhydratchemie gebräuchliche Schutzgruppe" eignet sich besonders ein Acetyl-, Benzoyl-, Benzyl- oder Trimethylsilyl-Rest.

Als Substituenten der Carboxamidogruppe kommen Alkyl-, Alkoxyalkyl-, Carboxyalkyl- und Alkoxycarbonylalkylreste in Frage, wobei die Alkylreste jeweils 1 bis 5, vorzugsweise 1 bis 3 Kohlenstoffatome aufweisen. Bei einer zweifach substituierten Carboxamidfunktion können die beiden Substituenten zu einem Ring geschlossen sein, der durch Heteroatome, z. B. Sauerstoff, Stickstoff und Schwefel, unterbrochen sein kann.

Als glycosidischer Rest, der mit den Resorufin-Derivaten der allgemeinen Formel II zu Glycosiden der allgemeinen Formel I verknüpft ist, eignen sich alle Mono- und Oligo-Saccharide, die sich durch die entsprechenden Glycosidasen wieder von dem Resorufin-Grundgerüst abspalten lassen. Als Beispiele für erfindungsgemäße Glycoside seien erwähnt: β-D-Galactopyranoside α-D-Galactopyranoside, β-D-Glucopyranoside, α-D-Glucopyranoside sowie α-D-Mannopyranoside.

Als glycosidischer Rest sind auch Oligo-Saccharid-Ketten geeignet, die sich von Saccharidketten spaltenden Enzymen bis auf die Stufe eines mono- bzw. Oligo-Saccharids abbauen lassen, das sich seinerseits mit den entsprechenden Glycosiden direkt von Resorufin-Grundgerüst abspalten läßt. Als solche Oligo-Saccharidketten werden insbesondere Ketten verstanden, die aus 2 bis 10, vorzugsweise aus 2 bis 7 Mono-Saccharid-Einheiten aufgebaut sind.

Zu den Resorufinen der Formel II gehören auch die neuen Verbindungen der allgemeinen tautomeren Formeln II'a und II'b

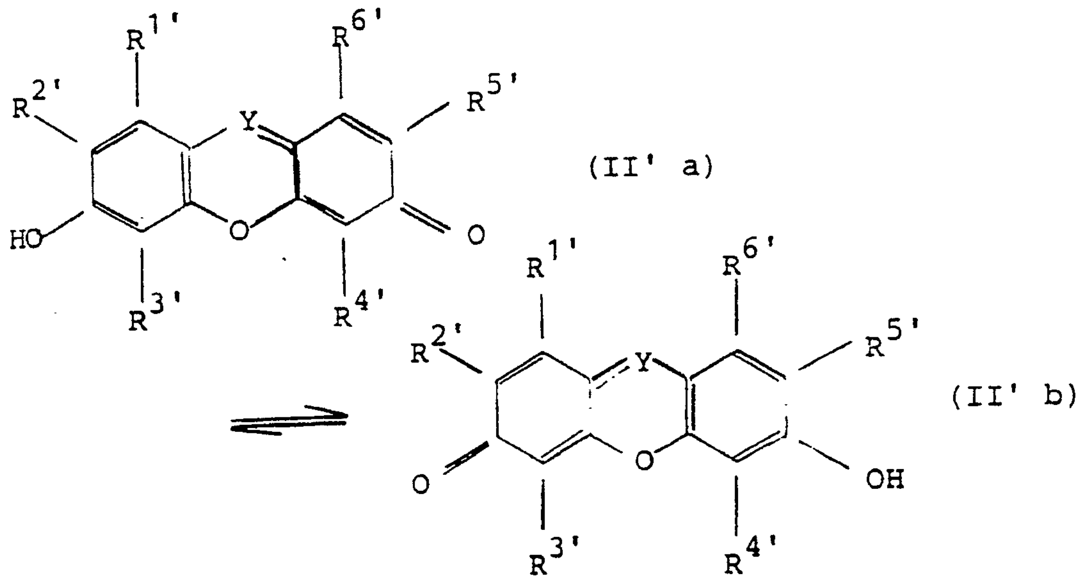

(II' a)

(II' b)

in denen

$R^{1'}$ bis $R^{6'}$ dieselbe Bedeutung wie die Substituenten $R^1$ bis $R^5$ besitzen, wobei $R^{1'}$ bis $R^{6'}$ nicht alle gleichzeitig Wasserstoff bedeuten können, und

Y die oben angegebene Bedeutung hat.

Diese Verbindungen sind neu. Sie eignen sich insbesondere als Zwischenprodukt zur Herstellung der erfindungsgemäßen Glycoside von Resorufin-Derivaten der allgemeinen Formel I.

Die Verbindungen der allgemeinen Formel II' lassen sich in Analogie zu Verfahren herstellen, die zur Herstellung des bekannten Resorufins (Verbindung der allgemeinen Formel II, in der $R^1$ bis $R^6$ jeweils ein Wasserstoffatom darstellt) geeignet sind.

Die Verbindungen der allgemeinen Formel II' werden in vorteilhafter Weise hergestellt, indem Nitrosoresorcin-Derivate der allgemeinen Formel III

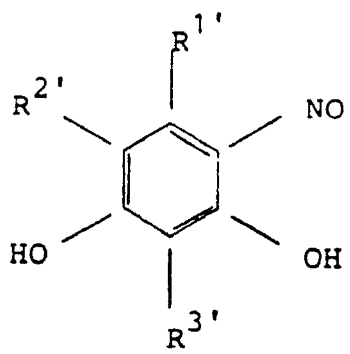

(III)

mit Resorcin-Derivaten der allgemeinen Formel IV

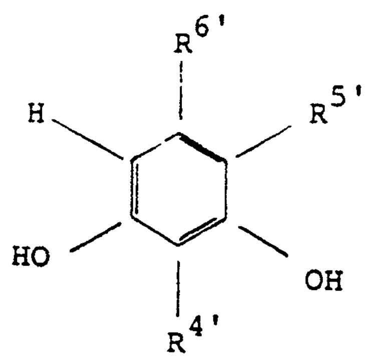

(IV)

in denen
$R^{1'}$ bis $R^{6'}$ die oben angegebene Bedeutung haben,
in Anwesenheit von Braunstein und Schwefelsäure bei niedrigen Temperaturen umgesetzt werden. Es entstehen dabei zunächst Verbindungen der allgemeinen Formel II', in der Y eine N→O-Gruppe darstellt. Diese Substanzen lassen sich leicht mit Zinkpulver in Anwesenheit von Ammoniak in Verbindungen der allgemeinen Formel II', in der Y Stickstoff bedeutet, überführen.

Die Reaktion der Verbindungen der allgemeinen Formel III mit Verbindungen der allgemeinen Formel IV wird üblicherweise bei einer Temperatur zwischen -10 bis 50° C, vorzugsweise zwischen 0 und 30° C durchgeführt. Besonders schonend verläuft die Umsetzung, wenn man die Substanzen der allgemeinen Formel III und der Formel IV bei ungefähr 0° C vermischt und das Reaktionsgemisch sich anschließend auf Raumtemperatur erwärmen läßt. Die Konzentration an Braunstein sollte zweckmäßigerweise bei 0,5 bis 5, vorzugsweise 1 bis 2 mol/l liegen. Die Schwefelsäurekonzentration sollte 0,5 bis 5, vorzugsweise 1 bis 3 mol/l betragen.

Die Reduktion von Verbindungen der allgemeinen Formel II', in der Y eine N→O-Gruppe darstellt, zu Verbindungen der allgemeinen Formel II', in der Y ein Stickstoff bedeutet, wird vorzugsweise in ammoniakalischer Lösung mit Zinkstaub durchgeführt (vgl. Nietzki et al., Ber. Dtsch. Chem. Ges. 22, 3020 [1889]). Als Lösungsmittel verwendet man zweckmäßigerweise Wasser-Alkohol-Gemische, bevorzugt ein Gemisch aus 1 Teil Wasser mit 0 bis 4 Teilen Methanol. Pro Mol zu reduzierender Substanz werden 1 bis 20, vorzugsweise 1 bis 5 Mol Zinkstaub portionsweise zugegeben. Die Temperatur der Reaktionslösung wird dabei bei -10 bis +35° C, vorzugsweise bei +5 bis +10° C gehalten. Die genaue Einhaltung des Temperaturbereiches hat sich als notwendig für einen eindeutigen Reaktionsverlauf erwiesen. Ohne Kühlung führt die exotherme Reaktion zu Nebenprodukten, die schwer abtrennbar sind.

Unter den gewählten milden Bedingungen verläuft die Umsetzung zwischen den Substanzen der allgemeinen Formel III und der allgemeinen Formel IV eindeutig und mit guter Ausbeute. Der gewählte Syntheseweg ist variationsfähig. Dies eröffnet insbesondere im Hinblick auf die Darstellung unsymmetrisch substituierter Resorufin-bzw. auch Resazurin- Derivate zahlreiche Synthesemöglichkeiten.

Ebenfalls Gegenstand der Erfindung sind Resorufin-Derivate der Formel II', in der $R^{4'}$ oder/und $R^{6'}$ eine Niederalkoxycarbonyl-, eine gegebenenfalls ein- oder zweifach substituierte Carboxamidogruppe oder die Gruppe $-COO-(CH_2CH_2O)_n-R^7$ bedeuten, Sie werden bevorzugt über die triacylierten Dihydroresorufine der allgemeinen Formel V dargestellt,

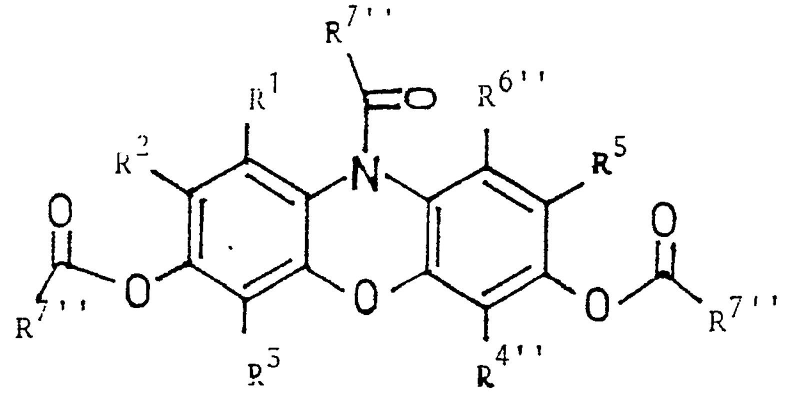

(V)

in der
$R^1$, $R^2$, $R^3$ und $R^5$ die oben angegebene Bedeutung haben,
$R^{4''}$ und $R^{6''}$ eine Carboxygruppe und
$R^{7''}$ Wasserstoff oder eine Niederalkylgruppe vorstellen.

Die Carbonsäure-Funktion wird durch literaturbekannte Verfahren, z. b. mit Oxalylchlorid/DMF oder Thionylchlorid/DMF in das Säurechlorid überführt, aus dem durch Reaktion mit beliebigen Alkoholen und Aminen die entsprechend substituierten Carbonsäureester- bzw. Carboxamid-Derivate erhalten werden.

Durch Behandlung der so erhaltenen acetylierten Derivate der allgemeinen Formel III mit Lauge, bevorzugt 0,1 - 5 M Natron- oder Kalilauge, oder mit 1 - 15 M wäßrigem Ammoniak und einem Oxidationsmittel, bevorzugt Kaliumhexacyanoferrat-III, unter Zugabe von wasserlöslichen organischen Lösungsmitteln, wie 1,4-Dioxan oder Methanol, erhält man die entsprechenden Resorufin-Derivate der allgemeinen Formel II.

Als Alkoholkomponente eignen sich im Prinzip alle möglichen Alkohole. Besonders bevorzugt sind Diethylenglycol-monoethyl-ether, Triethylenglycol-monoethyl-ether oder einfache Alkohole, wie Methanol oder Ethanol. Die Amin-Komponente kann ebenfalls aus allen möglichen Aminen ausgewählt werden.

Besonders bevorzugt sind Amine mit einer polaren Gruppe, wie beispielsweise Morpholin, Methoxyethylamin oder Glycinamid oder Ammoniak, ein primäres oder sekundäres Niederalkylamin. Ferner können Aminocarbonsäuren mit in üblicher Weise geschützter Carboxyl-Funktion, wie z. B. Glycin-tert-butyl-ester, Glycinbenzylester oder $N^{\alpha}$ -BOC-Lysinmethylester eingesetzt werden. Nach Abspaltung der Schutzgruppen erhält man so Resorufine II bzw. Resorufin-glycoside I mit aliphatischer Carbonsäure-Funktion.

Die acetylierten Dihydroresorufine der allgemeinen Formel V erhält man aus dem entsprechenden Resorufin oder Resazurin durch Umsetzung mit einem starken Reduktionsmittel, wie z. B. Zinn-II-chlorid oder Chrom-II-acetat oder durch elektrochemische Reduktion und anschließende Acetylierung. Zur Reduktion erwärmt man das Resorufin oder Resazurin 10 min bis 1 Std. mit 2 bis 10, bevorzugt 2 bis 6 Äquivalenten Zinn-II-chlorid in 5 bis 35 %iger wäßriger Salzsäure. Beim Abkühlen fällt die Dihydroverbindung aus. Die Acetylierung erfolgt in üblicher Weise mit Acetanhydrid. Bevorzugt werden die Verbindungen der allgemeinen Formel V in einem Eintopfverfahren durch reduktive Acetylierung hergestellt. Das entsprechende Resorufin oder Resazurin wird mit 2 bis 6 Äquivalenten Zinn-II-Chlorid 5 min bis 5 Std., bevorzugt 10 min bis 3 Std. in Acetanhydrid unter Rückfluß erhitzt oder bei RT 4 bis 16 Std. gerührt.

Bei der Synthese der Glycoside der Resorufin-Derivate der allgemeinen Formel II entstehen nicht-fluoreszierende Verbindungen, aus denen durch enzymatische Spaltung mit Hilfe der entsprechenden Glycosidasen wieder die fluoreszierenden Resorufin-Derivate freigesetzt werden. Die erfindungsgemäßen fluorogenen Resorufin-Derivate besitzen im Vergleich zu den bisher bekannten Glycosiden anderer chromogener bzw. fluorogener Gruppen sehr vorteilhafte Eigenschaften. Als monosubstituierte Derivate weisen sie eine Kinetik der enzymatischen Hydrolyse auf, die sehr einfach durch die Michaelis-Menten-Beziehung beschrieben werden kann. Für das Resorufin-$\beta$-D-galactopyranosid gilt beispielsweise eine Michaelis-Konstante $K_M$ = 0,38 mmol/l. Die Hemmung dieser Hydrolyse durch das natürliche Substrat Lactose ist kompetitiv, so daß für bestimmte Untersuchungen der spezifische Umsatz von Glycosiden der Resorufin-Derivate durch Zugabe von natürlichen Glycosiden, wie Lactose im Falle der $\beta$-D-Galaktosidase, definiert und reversibel verändert werden kann.

Die erfindungsgemäßen Glycoside von Resorufin-Derivaten sind in wäßriger Lösung bei +4° C praktisch unbegrenzt haltbar. Schon die Löslichkeit der Glycoside des Resorufin-Grundgerüstes ist für die meisten kinetischen Zwecke ausreichend. Durch Einführung von Carbonsäureresten, Carbonsäure-Derivaten mit polaren Gruppen bzw. Sulfonsäuregruppen wird die Löslichkeit der entsprechenden Glycoside noch

wesentlich verbessert.

Anregung und Emission der erfindungsgemäßen Produkte liegen im sichtbaren Spektralbereich bei hinreichender Quantenausbeute. Die maximale Fluoreszensintensität des Resorufins wird bei pH-Werten über 7,0 erreicht und fällt zu niedrigeren pH-Werten nur langsam ab. Die Glycoside des Resorufins sind in wäßriger Lösung meist gelb gefärbt ($\lambda_{max.}$ bei ca. 470 nm). Nach der enzymatischen Reaktion weisen die Produkte meist eine rote Farbe auf ($\lambda_{max.}$ bei ca. 570 nm), so daß die Substanzen ebenfalls ausgezeichnet für photometrische Bestimmungen und nicht-instrumentelle, visuelle Verfahren geeignet sind.

Ein weiterer Gegenstand der Erfindung ist die Verwendung der neuen Glycoside von Resorufin-Derivaten der allgemeinen Formel I zur Bestimmung der Aktivität von entsprechenden Glycosidasen, beispielsweise der $\alpha$-D- und $\beta$-D-Galactosidase, der $\alpha$-D- und $\beta$-D-Glucosidase sowie der $\alpha$-D-Mannosidase.

Resorufin-Derivate der allgemeinen Formel I, in denen der Glycosid-Rest eine Oligo-Saccharid-Kette darstellt, eignen sich insbesondere auch zum Nachweis von Saccharidketten spaltenden Enzymen, wie beispielsweise $\alpha$-Amylase. Hierbei wird die Oligo-Saccharidkette durch das nachzuweisende Enzym in charakteristische Weise gespalten, vorzugsweise bis zur Stufe des Monosaccharids, wobei erforderlichenfalls weitere Hilfsenzyme verwendet werden können. Dieses wird dann durch die entsprechende Glycosidase in vorstehend beschriebener Weise in das Resorufin-Grundgerüst und das Monosaccharid gespalten.

Ferner werden diagnostische Mittel zur Bestimmung der Aktivität von Glycosidasen beansprucht, die die neuen Glycoside von Resorufin-Derivaten der allgemeinen Formel I enthalten. Die Verwendung der Glycoside von Resorufin-Derivaten der allgemeinen Formel I als Substrate für die Glycosidasen führt zu deutlich empfindlicheren Testsystemen, als sie bisher bekannt sind. Die neuen Substrate können zur Bestimmung der Aktivität von Glycosidasen mit Vorteil sowohl im biochemischen, biotechnologischen als auch im klinisch-chemischen Bereich eingesetzt werden. Sie sind empfindlicher. Daraus ergeben sich mehrere Vorteile:

a) Es können geringere Enzym-Aktivitäten gemessen werden.

b) Es können kleinere Probenmengen eingesetzt werden.

c) Die Bestimmung der Aktivität kann in erheblich kürzerer Zeit erfolgen.

d) Der geringe Probeneinsatz und die günstige Meßwellenlänge vermindern außerdem die Störanfälligkeit der Methode durch andere Probenbestandteile.

e) Es kann der Umsatz in Trägermatrizen mit immobilisiertem Enzym gemessen werden.

Es hat sich gezeigt, daß die beanspruchten Substrate zur Bestimmung der Aktivität von Glycosidasen jeglicher Herkunft geeignet sind. Die erfindungsgemäßen diagnostischen Mittel mit Substraten der allgemeinen Formel I reagieren deutlich empfindlicher als die bisher bekannten Testmittel. Geeignet sind die Glycoside von Resorufin-Derivaten der Formel I auch für immunologische Bestimmungsmethoden, bei denen Glycosidasen als Indikator-Enzyme verwendet werden, deren Aktivität nach Durchführung der immunologischen Reaktion ermittelt werden muß. Solche immunologischen Bestimmungsmethoden mit enzymatischer Indikatorreaktion sind dem Fachmann als Enzymimmunoassays bekannt. Diese Methoden dienen zur Bestimmung der Konzentration von Proteinen, Polysacchariden, Hormonen, Arzneistoffen und anderen nieder-molekularen Substanzen im Bereich von $10^{-5}$ bis $10^{-12}$ mol/l. Je nach Erfordernis von Phasentrennschritten unterscheidet man zwischen homogener und heterogener Testführung. Eine weitere Unterteilung kann in kompetitive und nicht-kompetitive Testprinzipien erfolgen.

Alle Testprinzipien arbeiten jedoch mit Enzym-Antigen- bzw. Enzym-Antikörper-Konjugaten. Die enzymatische Indikatorreaktion ist allen Enzymimmunoassays gemeinsam. Für solche Zwecke geeignete Indikatorenzyme sind beispielsweise Glycosidasen, insbesondere die $\beta$-D-Galactosidase. Die Bestimmung der Glycosidasen in solchen Enzymimmunoassays erfolgt üblicherweise, indem ein geeignetes Substrat zugesetzt wird, das enzymatisch gespalten und in üblicher Weise photometrisch oder auch fluorometrisch vermessen wird.

Eine Verbesserung des Glycosidase-Testsystems führt demnach ebenfalls zu erheblichen Vorteilen bei solchen Enzymimmunoassays:

1. Die höhere Empfindlichkeit ermöglicht auch hier eine weitere Erniedrigung der Nachweisgrenzen, kürzere Reaktionszeiten und geringeren Probeneinsatz und damit auch geringere Störungen durch andere Probenbestandteile.

2. Die günstigere Meßwellenlänge vermindert bei bestimmten Reaktionsführungen die Störanfälligkeit der Methode durch unlösliche Bestandteile, beispielsweise durch Trübungen.

Das diagnostische Mittel enthält neben einem oder mehreren der erfindungsgemäßen Substrate der allgemeinen Formel I, ein geeignetes Puffersystem sowie gegebenenfalls weitere geeignete, üblicherweise für solche diagnostische Mittel verwendete Zusatzsstoffe, wie beispielsweise Netzmittel, Stabilisatoren usw. Das diagnostische Mittel kann in Form einer Lösung, als Lyophilisat, als Pulvergemisch, Reagenztablette oder auf einem saugfähigen Träger aufgezogen, vorliegen.

Das erfindungsgemäße diagnostische Mittel in Form einer Lösung enthält vorzugsweise sämtliche für den Test benötigten Reagentien. Als Lösungsmittel kommen Wasser oder Gemische von Wasser mit einem wasserlöslichen organischen Lösungsmittel, wie z. B. Methanol, Äthanol, Aceton oder Dimethylformamid, in Frage. Aus Haltbarkeitsgründen kann es vorteilhaft sein, die für den Test benötigten Reagentien auf zwei oder mehr Lösungen zu verteilen, die erst bei der eigentlichen Untersuchung vermischt werden.

Zur Herstellung des diagnostischen Mittels in Form eines Lyophilisats im Gesamtgewicht von jeweils etwa 5 - 20 mg, vorzugsweise etwa 10 mg, wird eine Lösung getrocknet, die neben sämtlichen für den Test benötigten Reagentien übliche Gerüstbildner, wie z. B. Polyvinylpyrrolidon, und eventuelle weitere Füllstoffe, wie z. B. Mannit, Sorbit oder Xylit enthält.

Ein diagnostisches Mittel in Form von Pulvermischungen oder Reagenztabletten läßt sich herstellen, indem die Bestandteile des Testes mit üblichen galenischen Zusatzstoffen versetzt und granuliert werden. Zusatzstoffe dieser Art sind z. B. Zuckeralkohole, wie Mannit, Sorbit oder Xylit, oder andere lösliche inerte Verbindungen, wie Polyethylenglykole oder Polyvinylpyrrolidon. Die Pulvermischungen oder Reagenztabletten weisen im allgemeinen ein Endgewicht von ungefähr 30 bis 200 mg, vorzugsweise 50 bis 80 mg, auf.

Zur Herstellung des diagnostischen Mittels in Form eines Teststreifens wird ein saugfähiger Träger, vorzugsweise Filterpapier, Cellulose oder Kunstfaservlies mit Lösungen der erforderlichen, üblicherweise zur Herstellung von Teststreifen verwendeten Reagentien in leicht flüchtigen Lösungsmitteln, wie z. B. Wasser, Methanol, Äthanol oder Aceton imprägniert. Dies kann in einem Imprägnierschritt erfolgen. Oft ist es jedoch zweckmäßig, die Imprägnierung in mehreren Schritten durchzuführen, wobei Lösungen eingesetzt werden, die jeweils einen Teil der Bestandteile des diagnostischen Mittels enthalten. So kann beispielsweise in einem ersten Schritt mit einer wäßrigen Lösung, die den Puffer und andere wasserlösliche Zusatzstoffe enthält, und dann in einem zweiten Schritt mit einer Lösung, die das Glycosidase-Substrat enthält, imprägniert werden. Die fertigen Testpapiere können als solche verwendet werden oder in an sich bekannter Weise an Griffen angeklebt oder vorzugsweise zwischen Kunststoffen und feinmaschigen Netzwerken gemäß DBP 2118455 eingesiegelt werden.

Die nachfolgenden Beispiele zeigen einige der zahlreichen Verfahrensvarianten, die zur Synthese der erfindungsgemäßen Verbindungen beschritten werden können sowie beispielhaft die Verwendung der neuen Glycoside von Resorufin-Derivaten zur Bestimmung der Aktivität von Glycosidasen. Sie sollen jedoch nicht eine Einschränkung des Erfindungsgegenstandes bedeuten.

## Beispiel 1

### Resorufin-β-D-galactopyranosid

a) Darstellung von Resorufin

Resorufin wird aus seinem Oxidationsprodukt Resazurin, das in guter Reinheit kommerziell erhältlich ist, in Anlehnung an die Beschreibung von Nietzki et al. (Ber. Dtsch. Chem. Ges. 22 (1889) Seite 3020 - 3038) dargestellt. Hierzu werden 10 g (43 mmol) Resazurin (Fluka, Buchs, Schweiz) in einem Becherglas mit 50 ml 25 %iger Ammoniaklösung, 25 ml 37 %iger Natriumhydrogensulfit-Lösung und 50 ml Wasser für 30 Minuten auf dem siedenden Wasserbad erhitzt. Es werden nochmals 20 ml der Ammoniaklösung, 7 ml der Hydrogensulfit-Lösung und 20 ml Wasser dazugegeben und weitere 30 Minuten erhitzt. Die Umwandlung kann am Farbumschlag von blau nach rot oder über Dünnschicht-Chromatographie beobachtet werden. Nachdem die Reaktion vollständig abgelaufen ist, werden 10 ml 32 %iger Salzlösung dazugegeben, bis pH 5 erreicht ist. Die Reaktionslösung wird für einen Tag im Kühlschrank stehen gelassen. Danach wird der bräunliche Niederschlag abgesaugt, mit eiskalter Salzsäurelösung, pH 4, gewaschen und bei 110° C getrocknet.
Ausbeute 7,8 g (36,7 mmol; 85 % d. Th.).

b) Glycosylierung von Resorufin zu Resorufin-β-D-galactoyranosid

Die Glycosylierung an der phenolischen Hydroxylgruppe über die Zwischenstufe des Tetraacetats gelang nach dem folgenden Verfahren. 2,13 g (10 mmol) feingepulvertes Resorufin, 4,12 g (10 mmol) Acetobromo-α-D-galactose (Sigma, München), 1,16 g (5 mmol) Silber-I-oxid (Fluka, Buchs, Schweiz), 5 g Calciumsulfat ($CaSO_4$. 1/2 $H_2O$; Drierit), einige Körnchen Jod und 50 µl Chinolin werden in 50 ml Methylenchlorid bei Raumtemperatur für 40 Stunden gerührt. 40 % des Resorufins werden dabei zu Resorufin-β-D-galactopyranosid-tetra-acetat umgewandelt. Die Kontrolle der Reaktion kann wiederum mit Hilfe der Dünnschicht-Chromatographie durchgeführt werden. Nach Abtrennen der festen Bestandteile über

Faltenfilter und Zentrifugation wird das Methylenchlorid im Rotavapor abgezogen. Das Resorufin-$\beta$-D-galactopyranosid-tetra-acetat bleibt als braungelblicher Sirup zurück. Ausbeute ca. 2 g (35 % d. Th.).

2 g Resorufin-$\beta$-D-galactopyranosid-tetra-acetat werden ohne weitere Reinigung in 80 ml trockenem Methanol aufgenommen. 0,5 g Natrium werden in kleinen Stücken, im Eisbad, in 20 ml trockenem Methanol aufgelöst. Unter Rühren im Eisbad werden 6 - 8 ml der Methoxidlösung innerhalb von 30 Minuten in Portionen von 1 ml zu der Resorufin-$\beta$-D- galactopyranosid-tetra-acetat-Lösung gegeben und die Deacetylierung über Dünnschicht-Chromatographie beobachtet. Das ausgefallene, orangefarbige Resorufin-$\beta$- D-galactopyranosid wird abgesaugt und mit wenig eiskaltem Methanol gewaschen. Ausbeute ca. 1 g Rohsubstanz (70 % d. Th.). Umkristallisation aus Methanol mit nachfolgender Trockung (nicht über 60° C) liefert 0,2 g reines Produkt. Alle Dünnschicht-Chromatographie-Untersuchungen zur Kontrolle des Reaktionsablaufes können mit dem System Kieselgel 60 (Merck, Darmstadt) und Fließmittel Methylenchlorid/Methanol (9/1) durchgeführt werden. Für dieses System gelten die folgenden $R_f$-Werte:

| | |
|---|---|
| Resorufin | 0,4 |
| Resazurin | 0,35 |
| Resorufin-ß-D-galactopyranosid-tetra-acetat | 0,9 |
| Resorufin-ß-D-galactopyranosid | 0,1. |

Beispiel 2

Resorufin-$\beta$-D-glucosid

2,13 g (10 mmol) Resorufin (hergestellt gemäß Beispiel 1 a), 4,11 g (10 mmol) Acetobromo-$\alpha$-D-glucose und 3,64 g (10 mmol) Hexadecyltrimethylammoniumbromid werden 4 Std. in einer Mischung von 11,5 ml 1 N Natronlauge und 50 ml Chloroform unter Rückfluß erhitzt. Danach wird zur Trockne eingedampft und mit Essigester digeriert. Die Essigester-Lösung wird erneut zur Trockne eingedampft. Es wird mit 20 ml Ethanol aufgenommen. Das aus dieser Lösung ausgefallene Tetraacetylresorufin-$\beta$-D-glucosid wird aus Methanol unter Zusatz von Aktivkohle umkristallisiert.

Zur Deacetylierung wird mit 10 ml Methanol aufgenommen. Nach 2 Std. Rühren bei Raumtemperatur wird das ausgefallene Produkt abgesaugt und getrocknet. Ausbeute: 50 mg.

1H-NMR ($[D]_6$-DMSO): $\delta$ = 3.15 - 3.80 (m, 6H); 4.5 - 6.5 (breit, 4H); 5.12 (d, J = 6,9 Hz, 1H); 6.29 (d, J = 2,0 Hz, 1H); 6.80 (dd, J = 9.6 und 2.0 Hz, 1H); 7.12 (dd,, J = 8.5 und 2,0 Hz, 1H); 7.16 (d, J = 2.0 Hz, 1H); 7.55 (d, J = 9,6 Hz, 1H); 7.80 (d, J = 8.5 Hz, 1H).

Beispiel 3

Resorufin-1-carbonsäuremethylester

50 g Nitrosoresorcin, 53,3 g 3,5-Dihydroxybenzoesäuremethylester und 28,0 g Braunstein werden in 500 ml Methanol gelöst bzw. suspendiert. Unter Eiskühlung werden bei 5 bis 10° C 34,4 ml konzentrierte Schwefelsäure zugetropft. Nach Entfernen des Eisbades wird noch zwei Stunden weitergerührt. Danach werden unter Kühlung 200 ml wäßrige Ammoniaklösung zugegeben. Der ausgefallene Niederschlag

wird über einen Glasfaserfilter abfiltriert. Zu dem Filtrat gibt man portionsweise bei 5 bis 10° C 10 g Zinkpulver. Bei Raumtemperatur wird so lange gerührt, bis die Reduktion vollständig ist (DC-Kontrolle, Essigester/Methanol 4:1 als Fließmittel, Reaktionszeit ca. 1,5 Std.). Am Rotationsverdampfer wird bei 25° C Badtemperatur auf 1/3 des Volumens eingeengt. Durch Ansäuern mit konzentrierter Salzsäure bis zum Farbumschlag unter Kühlung wird das gewünschte Produkt ausgefällt. Nach Waschen mit verdünnter Salzsäure und Trocknen im Vakuum über Calciumchlorid erhält man Resorufin-1-carbonsäuremethylester. Ausbeute: 30,9 g (36 % d. Th.).

1H-NMR: ($[D]_6$-DMSO) : $\delta$ = 3.98 (s, 3H); 6,47 (d, J = 2.2 Hz, 1 H); 6.85 - 6.95 (m, 2H); 7.09 (d, J = 2.2 Hz, 1H); 7.60 (d, J = 9.6 Hz, 1 H).

Fluoreszenz:

Absorption: $\lambda_{max}$ = 570 nm

Emission: $\lambda_{max}$ = 588 nm.

In analoger Weise erhält man aus

a) 3,5-Dihydroxybenzoesäure und Nitrosoresorcin über die Resazurin-1-carbonsäure Resorufin-1-carbonsäure
UV/VIS (0,1 M Kaliumphosphat-Puffer, pH 7,5): $\lambda_{max}$ = 569 nm

b) 4-O-Methyl-gallussäuremethylester und Nitrosoresorcin über 4-Methoxy-resazurin-1-carbonsäuremethylester 4-Methoxy-resorufin-1-carbonsäuremethylester
UV/VIS (0,1 M Kaliumphosphat-Puffer, pH 7,5):
$\lambda_{max}$ = 592 nm

c) 3,5-Dihydroxybenzoesäuremethylester und 4-Chlor-6-nitrosoresorcin über 8-Chlor-resazurin-1-carbonsäuremethylester 8-Chlor -resorufin-1-carbonsäuremethylester

d) 4-O-Methylgallussäuremethylester und 4-Brom-6-nitrosoresorcin über 8-Brom-4-methoxy-resazurin-1-carbonsäuremethylester 8-Brom-4-methoxy-resorufin-1-carbonsäuremethylester

e) 5-Nitroresorcin und Nitrosoresorcin über 1-Nitro-resazurin 1-Nitro-resorufin

f) Resorcin-5-sulfonsäure und Nitrosoresorcin über Resazurin-1-sulfonsäure Resorufin-1-sulfonsäure

Beispiel 4

Resazurin-4-carbonsäure

1,60 g (10,5 mmol) Nitrosoresorcin, 1,55 g (10,0 mmol) 2,6-Dihydroxybenzoesäure und 0,86 g (10 mmol) Braunstein werden in 20 ml Methanol aufgenommen und auf 0° C gekühlt. Dazu werden 1,06 ml konz. Schwefelsäure zugetropft. Es wird noch 2 Stunden ohne Kühlung weitergerührt. Das ausgefallene rote Produkt wird abfiltriert, mit Methanol gewaschen und getrocknet. Ausbeute: 2,3 g (85 % d. Th.).
UV/VIS (0,1 M Kaliumphosphatpuffer pH 7,5):
$\lambda_{max}$ = 614 nm ($\epsilon$ = 48 $cm^2$ $mol^{-1}$)
Nach Ansäuren:
$\lambda_{max}$ = 522 nm ($\epsilon$ = 32 $cm^2$ $mol^{-1}$)

Beispiel 5

Resorufin-4-carbonsäure

2,3 g Resazurin-4-carbonsäure (hergestellt nach Beispiel 4) werden in 20 ml Wasser und 5 ml 25 %igem Ammoniak gelöst. Zu der blauen Losung werden unter Eiskühlung 5 g Zinkstaub gegeben. Danach wird die Eiskühlung entfernt, so daß sich die Lösung allmälich auf Raumtemperatur erwärmt. Die Reduktion läßt sich leicht am Farbumschlag von blau nach dunkelviolett erkennen bzw. mit Hilfe der Dünnschicht-Chromatographie (Fließmittel: Methanol/Essigester 1:1) beobachten. Das überschüssige Zinkpulver wird abfiltriert. Die Reaktionslösung wird mit 5 ml Eisessig und konz. Salzsäure angesäuert. Das ausgefallene Produkt wird abfiltriert, mit verdünnter Salzsäure gewaschen und im Vakuum über Calciumchlorid getrocknet. Ausbeute : 1,8 g (82 % d. Th.).
UV/VIS: (0,1 M Kaliumphosphatpuffer, pH 7,5):
$\lambda_{max}$ = 579,4 nm ($\epsilon$ = 48,6 $cm^2$ $mol^{-1}$);
Nach Ansäuren:
$\lambda_{max}$ = 485,9 nm ($\epsilon$ = 34,7 $cm^2$ $mol^{-1}$).
Fluoreszenz:
Absorption: $\lambda_{max}$ = 579 nm
Emission: $\lambda_{max}$ = 593 nm.

Beispiel 6

1-Methylresorufin-4-carbonsäure

840 mg 2,6-Dihydroxy-4-methylbenzoesäure, 760 mg Nitrosoresorcin, 430 mg Braunstein und 0,53 ml Schwefelsäure werden analog Beispiel 4 zu 1-Methylresazurin-4-carbonsäure umgesetzt. Ausbeute: 0,8 g. Die so erhaltene 1-Methylresazurin-4-carbonsäure wird analog Beispiel 5 zu 1-Methylresorufin-4-carbonsäure reduziert.
Ausbeute: 0,4 g.
UV/VIS (0,1 M Kaliumphosphat-Puffer, pH 7,5): $\lambda_{max}$ = 571 nm.

Beispiel 7

Resorufin-4-carbonsäure-morpholid

1) N,O,O-Triacetyldihydroresorufin-4-carbonsäure

Variante a)

5 g (19,4 mmol) Resorufin-4-carbonsäure oder 5,3 g (19,4 mmol) Resazurin-4-carbonsäure werden in 100 ml 10 %iger Salzsäure 0,5 Std. mit 7 g (38 mmol) Zinn-II-chlorid unter Rückfluß erhitzt. Dabei färbt sich die Lösung grün. Man läßt erkalten, filtriert die ausgefallene Dihydroresorufin-4-carbonsäure unter Stickstoff ab und trocknet im Vakuum über Phosphorpentoxid. Das so erhaltene Rohprodukt wird 30 min mit 30 ml Acetanhydrid und 20 mg Natriumacetat unter Rückfluß erhitzt. Man gibt das Reaktionsgemisch auf 200 ml Eiswasser und rührt 14 Std. Der Niederschlag wird aus Ethanol/Wasser umkristallisiert. Man erhält 4,8 g (65 %) der gewünschten Verbindung.
Schmelzpunkt: 197 - 199° C
DC (Kieselgel, Fließmittel Chloroform/Methanol/Eisessig 9:1:0,1),
Rf = 0,33

Variante b)

5,1 g (20 mmol) Resorufin-4-carbonsäure werden in 20 ml Acetanhydrid mit 11 g (60 mmol) Zinn-II-chlorid 1 Std. bei 80° C gerührt. Man gibt auf 230 ml Eiswasser, rührt 1 Std., filtriert und arbeitet analog Variante a) auf.
Ausbeute: 5,4 g (71 %)

2) N,O,O-Triacetyldihydroresorufin-4-carbonsäurechlorid

3,85 g (10 mmol) N,O,O-Triacetyldihydroresorufin-4-carbonsäure werden mit 5,4 ml (60 mmol) Oxalylchlorid versetzt und auf -10° C gekühlt. Dazu gibt man einen Tropfen Dimethylformamid und läßt das Reaktionsgemisch unter Rühren auf Raumtemperatur erwärmen. Das Edukt löst sich dabei unter Gasentwicklung. Nach Beendigung der Gasentwicklung rührt man noch 0,5 Std., dampft ein, nimmt je 3mal mit 20 ml trockenen Methylenchlorid auf und dampft bis zur Trockne ein. Man erhält so 4 g Rohprodukt, das ohne weitere Reinigung weiterverarbeitet wird.
DC (Kieselgel, Fließmittel Chloroform/Methanol/Eisessig 9:1:0,1)
Rf = 0,42;
farbloser Fleck, der sich nach einigen Stunden rot färbt.

3) N,O,O-Triacetyldihydroresorufin-4-carbonsäure-morpholid

11,3 g (31,4 mmol) rohes Säurechlorid werden in 150 ml trockenem Methylenchlorid gelöst. Dazu tropft man 8,7 ml (63 mmol) Triethylamin und anschließend 3,3 ml (37,7 mmol) Morpholin. Man rührt noch 2 Std., wäscht die Lösung mit 1 %iger Citronensäure, Natriumhydrogencarbonat-Lösung und Wasser, trocknet die organische Phase über Mgnesiumsulfat und dampft ein. Der Rückstand wird aus Ethanol kristallisiert.
Ausbeute: 8,1 g (63 %), Schmelzpunkt: 133 - 135° C (Zersetzung)

4) Resorufin-4-carbonsäure-morpholid

3,7 g (9 mmol) Triacetyldihydroresorufin-4-carbonsäuremorpholid werden mit 250 ml Methanol und 250 ml Wasser aufgenommen. Dazu gibt man 36 ml 1 n Natronlauge und 6,0 g (18 mmol) Kaliumhexacyanoferrat-III und rührt 14 Std. bei Raumtemperatur. Nach Ansäuern mit Salzsäure auf pH 3 dampft man zur Trockne ein und digeriert mit Aceton. Die Farbstoff-Lösung wird über 500 ml Kieselgel mit Aceton als Fließmittel filtriert. Nach Eindampfen des farbstoffhaltigen Eluats erhält man 2,3 g (80 %) Produkt.
UV/VIS (0,1 M Kaliumphosphat-Puffer, pH 7,5):
$\lambda_{max}$ = 575 nm, $\epsilon$ = 55.000 $cm^2$ $mol^{-1}$.
DC (Fließmittel, siehe unter 2)
Rf = 0,52.

$^1$H-NMR ([D]$_6$-DMSO):$\delta$ = 3,3 - 3,8 (m, 8 H); 6,50 (d, J = 2 Hz, 1 H); 6,64 (d, J = 10 Hz, 1 H); 6,76 (dd, J = 10 und 2 Hz, 1 H); 7,44 und 7,51 (jeweils d, J = 10 Hz, 2 H).

Beispiel 8

Tetraacetylresorufin-1-carbonsäuremethylester-$\beta$-D-galactopyranosid und Tetraacetylresorufin-9-carbonsäuremethylester-$\beta$-D-galactopyranosid

6,8 g Resorufin-1-carbonsäuremethylester (hergestellt nach Beispiel 3), 5,75 g Silber-I-oxid, 6,75 g Silbercarbonat, 15 g Molekularsieb 4 A und 10 g $\alpha$-Brom-tetraacetylgalactose werden mit 250 ml absolutem Chloroform 4 Std. bei Raumtemperatur gerührt. Nach Zugabe von weiteren 5 g $\alpha$-Brom-tetraacetylgalactose wird über Nacht weitergerührt. Die Reaktionsmischung wird über einen Glasfaserfilter filtriert und eingedampft. Das ölige Rohprodukt wird an 2 l Kieselgel mit Chloroform/Essigester 2:1 als Eluent chromatographiert. Man eluiert 2,5 g einer gelben Fraktion mit Rf = 0,28 (HPTLC Kieselgel, dasselbe Fließmittel). Durch Rühren mit Methanol wird Tetraacetylresorufin-9-carbonsäuremethylester-$\beta$-D-galactopyranosid in Form orangefarbener Kristalle erhalten.
Ausbeute 1,5 g.
$^1$H-NMR ([D]$_6$-DMSO): $\delta$= 1.95, 2.03, 2.04 und 2.14 (je s, 12 H); 3.88 (s, 3H); 4.11 (d, J = 7 Hz, 2H); 4.51 (t, J = 7 Hz, 2 H); 5.24 (m, 2 H); 5.36 (m, 1H); 5.69 (m, 1H); 6.31 (d, J = 2 Hz, 1H); 6.97 (d, J = 2 Hz, 1H); 7.04 (dd, J = 8.8 und 2.4 Hz, 1H); 7.14 (d, J = 2.4 Hz, 1H); 7.78 (dd, J = 8.8 Hz, 1H).

Danach werden 1,6 g einer ebenfalls gelben Fraktion mit Rf = 0,24 eluiert. Aus Methanol kristallisiert Tetraacetylresorufin-1-carbonsäuremethylester-$\beta$-D-galactopyranosid in Form gelboranger Kristalle.
Ausbeute: 1,2 g.
$^1$H-NMR ([D]$_6$-DMSO): $\delta$ = 1.95, 2.02, 2.04 und 2.14 (je s, 12H); 3.90 (s, 3 H); 4.09 (m, 2H); 4.51 (m, 1H); 5.24 (d, J = 7 Hz, 1H); 5.25 (m, 1H); 5.36 (m, 1H); 5.71 (m, 1H); 6.50 (d, J = 2 Hz, 1H); 6.83 (dd, J = 10 und 2Hz, 1H); 7.20 und 7.24 (je d, J = 2Hz, 2H); 7.47 (d, J = 10 Hz, 1H).

Zwischen den reinen Produkten läßt sich noch eine Mischfraktion eluieren aus der sich durch Umkristallisieren aus Methanol 2,5 g Kristalle eines Gemisches der beiden isomeren Verbindungen erhalten lassen.

In analoger Weise erhält man aus $\alpha$-Bromtetraacetylgalactose und

a) 4-Methoxy-resorufin-1-carbonsäuremethylester Tetraacetyl-4-methoxy-resorufin-1-carbonsäuremethylester -$\beta$-D-galactopyranosid und Tetraacetyl-6-methoxy-resorufin-9-carbonsäuremethylester-$\beta$-D-galactopyranosid.

b) 8-Chlor-resorufin-1-carbonsäuremethylester Tetraacetyl-8-chlor-resorufin-1-carbonsäuremethylester-$\beta$ -D-galactopyranosid und Tetraacetyl-2-chlor-resorufin-9-carbonsäuremethylester-$\beta$ -D-galactopyranosid.

Beispiel 9

Tetraacetylresorufin-4-carbonsäure-morpholid-$\beta$-D-galactopyranosid und Tetraacetylresorufin-6-carbonsäure-morpholid-$\beta$-D-galactopyranosid

3,26 g (10 mmol) Resorufin-4-carbonsäuremorpholid werden analog Beispiel 8 galactosidiert. Das Rohprodukt wird an 1 l Kieselgel mit Essigester/Aceton 3:1 als Fließmittel chromatographiert. Man erhält so 0,9 g Tetraacetylresorufin-6-carbonsäuremorpholid-$\beta$-D-galactopyranosid,
DC (Kieselgel, Fließmittel siehe Beispiel 7)
Rf = 0,71,
und 0,4 g Tetraacetylresorufin-4-carbonsäuremorpholid-$\beta$-D-galactopyranosid,
DC (Kieselgel, dasselbe Fließmittel)
Rf = 0,76
sowie 1,2 g einer Mischfraktion aus beiden Isomeren.

Beispiel 10

Resorufin-1-carbonsäuremethylester-$\beta$-D-galactopyranosid

1,2 g Tetraacetylresorufin-9-carbonsäuremethylester-$\beta$-D-galactopyranosid werden analog Beispiel 2 mit Natriummethylat/Methanol deacetyliert. Ausbeute: 0,8 g.
UV/VIS (0,1 M Kaliumphosphat-Puffer, pH 7,5):

$\lambda_{max}$ = 464 nm ($\epsilon$ = 21,8 $cm^2$, $mol^{-1}$).

Nach Spaltung mit β-Galactosidase erhält man das Anion des Resorufin-1-Carbonsäuremethylesters: $\lambda_{max}$ = 572 nm ($\epsilon$ = 65,4 $cm^2$ $mol^{-1}$).

$^1$H-NMR ($[D]_6$-DMSO):δ = 3.20 - 3.80 (m, 6H); 3.91 (s, 3H); 5.09 (d, J = 7.5 Hz, 1H); 6.30 (d, J = 2.1 Hz, 1H); 6.81 (dd, J = 9.8 und 2.1 Hz, 1H); 7.30 (m, 2H); 7.51 (d, J = 9.8 Hz, 1H); OH-Protonen sehr breit um 5.

In analoger Weise erhält man durch Deacetylierung der entsprechenden Tetraacetate

a) Resorufin-1-carbonsäuremethylester-β-D-galactopyranosid

$^1$H-NMR ($[D]_6$-DMSO):δ = 3,4 - 3,7 (m, 6 H); 5,09 (d, J = 7,5 Hz, 1 H); 6,34 (d, J = 2 Hz, 1 H); 6,97 (d, J = 2 Hz, 1 H); 7,08 - 7,17 (m, 2 H); 7,75 (d, J = 10 Hz, 1 H); OH: sehr breit, um 5 ppm.

b) 4-Methoxy-resorufin-1-carbonsäuremethylester-β-D-galactopyranosid

c) 6-Methoxy-resorufin-9-carbonsäuremethylester-β-D-galactopyranosid

d) 8-Chlor-resorufin-1-carbonsäuremethylester-β-D-galactopyranosid

e) 2-Chlor-resorufin-9-carbonsäuremethylester-β-D-galactopyranosid

f) Resorufin-6-carbonsäuremorpholid-β-D-galactopyranosid

Rf (Essigester/Isopropanol/Wasser 9:4:2):0.3

Nach Spaltung mit β-Galactosidase:

UV/VIS (0.1 m Kaliumphosphatpuffer, pH 7.5):

$_{max}$ = 574,6 nm

Fluoreszenzemission: $_{max}$ = 593 nm

g) Resorufin-4-carbonsäuremorpholid-β-D-galactopyranosid

Rf (Essigester/Isopropanol/Wasser 9:4:2):0.3

Nach Spaltung mit β-Galactosidase:

UV/VIS (0.1 m Kaliumphosphatpuffer, pH 7.5):

$_{max}$ = 574,6 nm

Fluoreszenzemission: $_{max}$ = 593 nm

## Beispiel 11

## Resorufin-9-carbonsäure-β-D-galactopyranosid-triethylammoniumsalz

266 mg Resorufin-9-carbonsäuremethylester-β-D-galactopyranosidwerden in 50 ml Wasser und 20 ml 1,4-Dioxan aufgenommen. Dazu werden in Portionen von 0,5 ml 5 ml 0,1 N Natronlauge zugegeben, wobei der pH-Wert der Lösung nicht über 12,5 steigen darf. Das Reaktionsgemisch wird auf 6 ml DEAE-Sephadex, Carbonat-Form, gegeben. Es wird mit 180 ml Wasser gewaschen.

Danach wird das Produkt mit 0,1 M Triethylammoniumcarbonat-Puffer, pH 7,5 eluiert. Das Eluat wird im Vakuum eingedampft. Danach wird mehrmals mit Ethanol abgedampft. Ausbeute: 110 mg Resorufin-9-carbonsäure-β-D-galactopyranosid-triethylammoniumsalz.

UV/VIS (0,1 M Kaliumphosphat-Puffer, pH 7,5):

$\lambda_{max}$ = 465 nm

Nach Spaltung mit β-Galactosidase:

$\lambda_{max}$ = 570 nm

## Beispiel 12

## Resazurin-β-D-galactopyranosid

12,6 g Resazurin-Natriumsalz werden in 65 ml 1 N Natronlauge und 80 ml Wasser gelöst. Dazu werden 20,6 g Acetobrom-α-D-galactose und 18,2 g Hexadecyltrimethylammoniumbromid in 150 ml Chloroform gegeben. Die Mischung wird 3 Std. unter Rückfluß erhitzt. Danach wird zur Trockne eingedampft, und mit Essigester digeriert. Die Essigester-Lösung wird wieder eingeengt und an 500 g Kieselgel chromatographiert (Eluent: Methylenchlorid/Essigester 3:1). Es werden 2,13 g Tetraacetylresazurin-β-D-galactopyranosid mit Rf = 0,5 (HPTLC Kieselgel, dasselbe Fließmittel) erhalten.

Zur Deacetylierung werden 2,13 g Tetraacetyl-Derivat mit 0,1 g Natriummethylat in 100 ml absolutem Methanol 1 Std. bei Raumtemperatur gerührt. Das ausgefallene Produkt wird abgesaugt und über Calciumchlorid im Vakuum getrocknet.

Ausbeute: 1,0 g.

Rf = 0,62 (HPTLC Kieselgel, Essigester/Isopropanol/Wasser 9:4:2).

$^1H$-NMR ($[D]_6$-DMSO): $\delta$ = 3.30 - 3.90 (m, 6H); 4.54 (br.d, J = 4.4 Hz, 1H); 4,67 (br. t, J = 4.4 Hz, 1H); 5.07 (d, J = 7 Hz, 1H); 5.27 (br. d, J = 4.4 Hz, 1H); 6.15 (d, J = 2 Hz, 1H); 6.63 (dd, J = 9.6 und 2 Hz, 1H); 7.10 (dd, J = 9 und 2 Hz, 1H); 7.2 (m, 2H); 7.96 (d, J = 9.6 Hz, 1H); 8.07 (d, J = 9 Hz, 1H).

Beispiel 13

8-Chlor-resazurin-4-carbonsäure

4,3 g (30 mmol) 4-Chlor-resorcin werden in 20 ml Ethanol gelöst. Nach Zugabe von 2,4 g Kaliumhydroxyd, wird auf 5° C gekühlt. Unter Kühlung werden 2,81 ml Isopentylnitrit zugetropft. Danach wird über Nacht bei Raumtemperatur weitergerührt. Der Niederschlag wird abfiltriert, in Wasser aufgelöst und angesäuert. Der gelbe Niederschlag wird erneut filtriert und bei 40° C im Vakuum getrocknet. Ausbeute: 2,5 g ( 48 %) 4-Chlor-6-nitrosoresorcin
$R_f$: 0,28 (HPTLC, Kieselgel,
Fließmittel: Methanol/Essigester 1:3).

1,83 g 4-Chlor-6-nitrosoresorcin, 1,55 g 2,6-Dihydroxybenzoesäure, 0,86 g Braunstein und 1,06 ml Schwefelsäure werden in 20 ml Methanol gegeben und 2 Stunden bei 0° C sowie 14 Stunden bei Raumtemperatur gerührt. Man erhält einen roten Niederschlag, der abfiltriert und getrocknet wird.
Ausbeute: 2,45 g (80 %) 8-Chlor-resazurin-4-carbonsäure.
UV/VIS (0,1 M Kaliumphosphat-Puffer, pH 7,5):
$\lambda_{max}$ = 621,5 nm.

In analoger Weise erhält man aus
a) 2-Methyl-resorcin über das
2-Methyl-6-nitrosoresorcin
6-Methyl-resazurin-4-carbonsäure
b) 4-Methyl-resorcin über das 4-Methyl-6-nitrosoresorcin
8-Methyl-resazurin-4-carbonsäure
c) 4-Brom-resorcin über das 4-Brom-6-nitrosoresorcin
8-Brom-resazurin-4-carbonsäure.

Beispiel 14

8-Chlor-resorufin-4-carbonsäure

2 g 8-Chlor-resazurin-4-carbonsäure (hergestellt nach Beispiel 10) werden in 20 ml Wasser und 5 ml 25 %igen Ammoniak gelöst. Unter Eiskühlung wird Zinkpulver bis zum vollständigen Farbumschlag nach rotviolett zugegeben. Vom überschüssigen Zink wird abfiltriert. Nach dem Ansäuern wird das ausgefallen Produkt abfiltriert und im Vakuum bei 40° C über Kalziumchlorid getrocknet. Ausbeute: 1,5 g (79 %) 8-Chlor-resorufin-4-carbonsäure.
UV/VIS (0,1 M Kaliumphosphat-Puffer, pH 7,5):
$\lambda_{max}$ = 585,5 nm.

In analoger Weise erhält man aus
a) 6-Methyl-resazurin-4-carbonsäure
6-Methyl-resorufin-4-carbonsäure,
b) 8-Methyl-resazurin-4-carbonsäure
8-Methyl-resorufin-4-carbonsäure,
c) 8-Brom-resazurin-4-carbonsäure
8-Brom-resorufin-4-carbonsäure.

Beispiel 15

Resorufin-9-carbonsäuremethylester-α-D-galactopyranosid

3,9 g Pentaacetylgalactose werden mit 0,1 g wasserfreiem Zinkchlorid versetzt und auf 125° C erhitzt. Es wird 20 Minuten bei 10 Torr gerührt. Zu der Schmelze werden 1 g Resorufin-1-carbonsäuremethylester gegeben. Die Mischung wird 1 Stunde bei 41° C gerührt und danach in der in Beispiel 7 beschriebenen Weise an Kieselgel mit Chloroform/Essigester 2:1 chromatographiert.
Ausbeute: 25 mg Tetraacetyl-resorufin-9-carbonsäuremethylester-α-D-galactopyranosid (gelbes Öl)

Rf = 0,22 (HPTLC, Kieselgel, dasselbe Fließmittel).

Die Deacetylierung zu Resorufin-9-carbonsäuremethylester-α-D-galactopyranosid wird analog Beispiel 10 ausgeführt.

Beispiel 16

Resorufin-9-carbonsäure-(3.6-dioxaoctyl)-ester-β-D-galactopyranosid

0,6 g Tetraacetylresorufin-9-carbonsäuremethylester-β-D-galactopyranosid werden mit 50 ml Diethylenglycolmonoethylether und einer Spatelspitze Natriumhydrid versetzt und 15 Minuten bei Raumtemperatur gerührt. Danach werden 100 ml Aceton zugegeben. Der ausgefallene Niederschlag wird abfiltriert und getrocknet. Ausbeute: 56 mg Resorufin-9-carbonsäure-(3.6-dioxaoctyl)-ester-β-D-galactopyranosid
Rf: 0,54 (Kieselgel HPTLG, Fließmittel: Essigester/Isopropanol 9:4)

Beispiel 17

Resorufin-maltoheptaosid

Amylase (E.C. 2.4.1.19), beispielsweise Amylase aus Bacillus macerans hat neben hydrolytischer und cyclisierender Wirkung auch glycosyl-transferierende Eigenschaften, die zur Synthese von Oligosacchariden und -derivaten ausgenützt werden können (Methods in Carbohydrate Chemistry II, 347).

| | |
|---|---|
| 680 mg | Amylase aus Bacillus macerans DSM 24 (Lyophilisat; 0,46 U/mg Einwaage; Proteingehalt der Einwaage 28,5 %), |
| 500 mg | Resorufin-glucosid, |
| 3,5 g | α-Cyclodextrin und |
| 70 ml | Soerensen Phosphat-Puffer (pSH 6.2; 0,01 M) |

werden gemischt. Der Ansatz wird 24 Stunden lang bei 37° C inkubiert. Zur Aufreinigung wird dann α- und entstandenes β-Cyclodextrin zunächst über die Tetrachloräthyleneinschlußverbindung abgetrennt. Nach Chromatographie an vernetztem Dextran (Sephadex LH 20) erhält man 50 mg Lyophilisat von im Amylasetest hochaktivem Resorufinylmaltoheptaosid.

Beispiel 18

Bestimmung der Aktivität der β-D-Galactosidase

a) Zubereitung der verwendeten Lösungen:

| | | |
|---|---|---|
| Pufferlösung: | HEPES | 100 mmol/l |
| | Natriumchlorid | 154 mmol/l |
| | Magnesium-L-Aspartat | 2 mmol/l |
| | BSA | 10 g/l |
| | Tween-20 | 0,5 g/l |
| | pH-Wert (mit Natronlauge eingestellt) | 7,3 (37° C) |

Reagenslösung 1:

In der vorstehend beschriebenen Pufferlösung werden 0,8 mmol/l Resorufin-β-D-galactopyranosid gelöst.

Reagenslösung 2:

In der vorstehend beschriebenen Pufferlösung werden 3,5 mmol/l Resorufin-9-carbonsäure-β-D-galactopyranosid gelöst.

Reagenslösung 3:

In der vorstehend beschriebenen Pufferlösung werden 1,0 mmol/l Resorufin-9-carbonsäuremethylester-β-D-galactopyranosid gelöst.

Enzymlösung

Handelsübliche β-D-Galactosidase aus Escherichia coli wird in der vorstehend genannten Pufferlösung gelöst. Die Aktivität dieser Lösung beträgt ca. 0,08 U/ml (bezogen auf die Hersteller-Angaben).

b) Durchführung der Messungen

Die Messung erfolgt photometrisch bei 579 nm.

950 µl Reagens werden in einer 1-cm-Küvette bei 37° C mit 50 µl Enzymlösung vermischt. Als Maß für die Reaktion wird der Extinktionsanstieg pro Zeiteinheit in [mExt/min] ermittelt. Er wird berechnet aus der gemessenen Extinktion durch Division mit der Reaktionszeit.

In der folgenden Tabelle sind die gefundenen Meßwerte aufgeführt:

| Reagens Nr. | Reaktion [mExt/min] |
|---|---|
| 1 | 85 |
| 2 | 46 |
| 3 | 76 |

Beispiel 19

Nachweis von β-D-Galactosidase mit Hilfe eines Indikatorfilms

Zu einer homogenen Masse werden verarbeitet:
5 ml einer wäßrigen Lösung mit 0,5 M Kaliumphosphat und
0,05 M Magnesiumchlorid, pH 7,3 .
0,13 g Natriumalginat
8 g einer 50 %igen Dispersion von Polyvinylpropionat
10 g Kieselgel
12 ml Wasser
0,4 g Triton X-100
50 mg Resorufin-β-D-galactopyranosid, in 5 ml Methanol gelöst.

Diese Masse wird auf eine 0,1 mm starke Polycarbonatfolie (Pokalon, Fa. Lonza) mit einer Spaltbreite von 0,2 mm aufgetragen. Die Beschichtung wird bei 50° C getrocknet und danach in Stücke von 6 auf 6 mm geschnitten. Diese werden mit Hilfe von Klebeband auf eine 400 µm starke Polystyrolfolie aufgeklebt.

Die so erhaltenen Teststreifen werden kurze Zeit in die zu prüfende, β-D-Galactosidase enthaltende Lösung getaucht. Nach einer Wartezeit von 2 Minuten bei Raumtemperatur hat sich eine rote Reaktionsfarbe herausgebildet, deren Intensität von der Konzentration der β-D-Galactosidase in der Testlösung abhängt. Mit Hilfe von Testlösungen mit einem bestimmten, bekannten β-D-Galactosidase-Gehalt kann man eine Farbskala erhalten, anhand derer der unbekannte Gehalt an β-D-Galactosidase in einer Probe ermittelt werden kann.

Die vorstehend beschriebenen Teststreifen lassen sich auch dazu benutzen, die in einer Probe vorhandene β-D-Galactosidase Aktivität kinetisch mit Hilfe der Reflexionsphotometrie zu bestimmen. Auch hierzu wird in üblicher Weise anhand von Proben mit bekannter β-D-Galactosidase-Aktivität eine Eichkurve erstellt, mit deren Hilfe eine unbekannte β-D-Galactosidase-Aktivität einer Probe ermittelt werden kann.

Beispiel 20

Bestimmung der Aktivität freier und konjugierter β-D-Galactosidase

a) Herstellung der verwendeten Lösungen

| | | |
|---|---|---|
| Pufferlösung: | HEPES | 100 mmol/l |
| | Natriumchlorid | 154 mmol/l |
| | Magnesium-L-Aspartat | 2 mmol/l |
| | BSA | 10 g/l |
| | Tween-20 | 0,5 g/l |
| | pH-Wert (mit Natronlauge eingestellt) | 7,3 (37° C) |

Reagenslösung:

In vorstehend beschriebener Pufferlösung werden 0,8 mmol/l Resorufin-β-D-galactopyranosid gelöst.

Enzymlösung:

Handelsübliche β-D-Galactosidase aus Escherichia coli wird in Puffer gelöst. Die Aktivität dieser Lösung beträgt ca. 0,08 U/ml (bezogen auf die Hersteller-Angaben).

Enzym-Konjugat-Lösung:

Verwendet wird ein β-D-Galactosidase-Antikörper-Präparat. Die Herstellung solcher Enzym-Antikörper-Konjugate ist bekannt. Sie ist beispielsweise in Biochem. Biophys. Acta 612, 40 - 49 (1980) beschrieben. Das Präparat wird in Puffer so verdünnt, daß sich eine mit der oben beschriebenen Enzymlösung ungefähr vergleichbare Aktivität ergibt.

b) Durchführung der Messung:

Die Messung erfolgt photometrisch bei 578 nm. 950 µl Reagenslösung werden jeweils in einer 1-cm-Küvette bei 37°C mit 50 µl Enzymlösung bzw. mit 50 µl Enzym-Konjugat-Lösung vermischt. Als Maß für die Reaktion wird der Extinktionsanstieg pro Zeiteinheit in [mExt/min] ermittelt.

Für die Reaktion mit der freien β-D-Galactosidase werden 85 mExt/min; für die Reaktion mit β-D-Galactosidase-Antikörper-Konjugat 92 mExt/min gemessen.

Beide Meßwerte zeigen, daß sowohl mit freier als auch mit konjugierter β-D-Galactosidase ein sehr gut meßbarer Extinktionsunterschied gefunden wird.

Daraus ergibt sich, daß die beschriebenen Verbindungen als Substrate sowohl für freie Glycosidase als auch für Glycosidase-Konjugate in gleicher Weise geeignet sind. Die neuen Substrate können somit nicht nur als diagnostische Mittel für die Bestimmung freier Glycosidasen eingesetzt werden. Sie sind in vorteilhafter Weise auch bei immunologischen Bestimmungsmethoden einsetzbar, bei denen eine Glycosidase als Indikator-Enzym verwendet wird.

Beispiel 21

Bestimmung der α-Amylase-Aktivität

Filterpapier wird mit Resorufin-maltoheptaosid (hergestellt gemäß Beispiel 17), gelöst in Citratpuffer, pH 6, getränkt. Das so erhaltene Reagenzpapier wird nacheinander mit der α-Amylase enthaltenden Probe sowie mit α- und β-Glucosidase versetzt. Nach wenigen Minuten ist eine deutlich sichtbare Rotfärbung

nachzuweisen, deren Intensität oder -Amylase-Konzentration in der Probe proportional ist.

Mit Hilfe von Proben mit bekannter α-Amylase-Konzentration läßt sich eine Eichkurve ermitteln, anhand derer der unbekannte α-Amylase-Gehalt einer Probe bestimmt werden kann.

In den vorstehenden Beispielen wurden die folgenden Abkürzungen verwendet:

| | |
|---|---|
| HEPES | 2-[4-(2-Hydroxyethyl)-1-piperazinyl]-ethansulfonsäure |
| BSA | Rinderserum-Albumin, (bovineserum-albumin) |
| Tween 20 | Polyoxyethylen(20)sorbitanmonolaurat |

**Patentansprüche**

**1.** Glycoside von Resorufin-Derivaten der allgemeinen Formeln Ia bzw. Ib

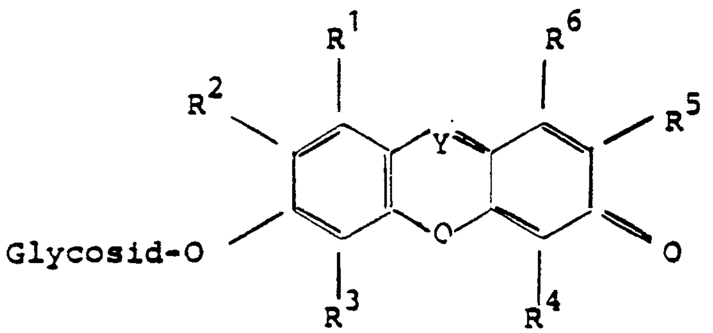

(I a)

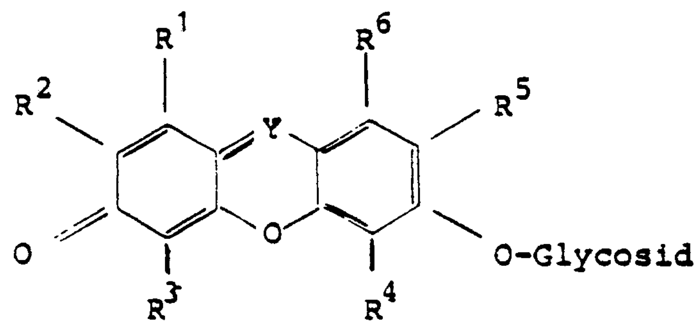

(I b)

in denen

$R^1$ Wasserstoff,
$R^2$, $R^3$ und $R^5$, die gleich oder verschieden sein können, Wasserstoff, Halogen oder eine $C_1$-$C_5$-Alkylgruppe
$R^4$ und $R^6$, die gleich oder verschieden sein können, Wasserstoff, Halogen, eine Cyano-, $C_1$-$C_5$- Alkyl-, $C_1$-$C_5$-Alkoxy-, Carboxy-, $C_1$-$C_5$-Alkoxycarbonyl-, Carboxy-$C_1$-$C_5$-alkyl-, $C_1$-$C_5$- Alkoxycarbonyl-$C_1$-$C_5$-alkyl- oder eine gegebenenfalls ein- oder zweifach substituierte Carboxamidogruppe oder die Gruppe

$-COO-(CH_2CH_2O)_n-R^7$

in der $R^7$ Wasserstoff oder eine $C_{1-5}$-Alkyl-Gruppe
und n eine ganze Zahl von 1 bis 4 bedeuten,
wobei $R^6$ zusätzlich eine Sulfonyl- oder Nitrogruppe vorstellen kann, und
Y Stickstoff oder die Gruppe N→O

bedeuten.

**2.** Glycoside gemäß Anspruch 1, dadurch gekennzeichnet, daß der Glycosid-Rest ein β-D-Galactopyranosid-, α-D-Galactopyranosid-, β-D-Glucopyranosid-, α-D-Glucopyranosid oder α-D-Mannopyranosid-Rest vorstellt.

**3.** Glycoside gemäß Anspruch 1, dadurch gekennzeichnet, daß der Glycosid-Rest ein Oligosaccharid mit

2 bis 10 Mono-Saccharid-Einheiten vorstellt.

4. Verfahren zur Herstellung der Glycoside von Resorufin-Derivaten der allgemeinen Formeln Ia bzw. Ib,

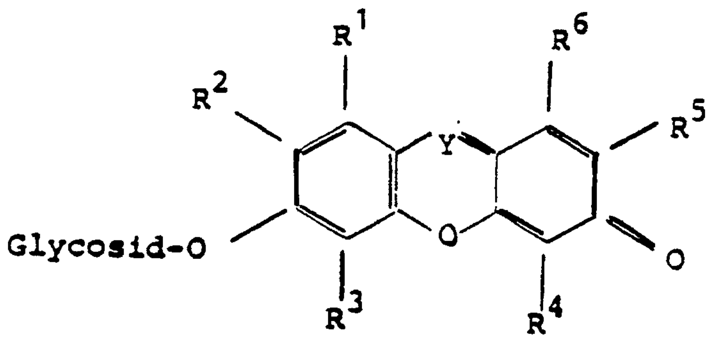

(I a)

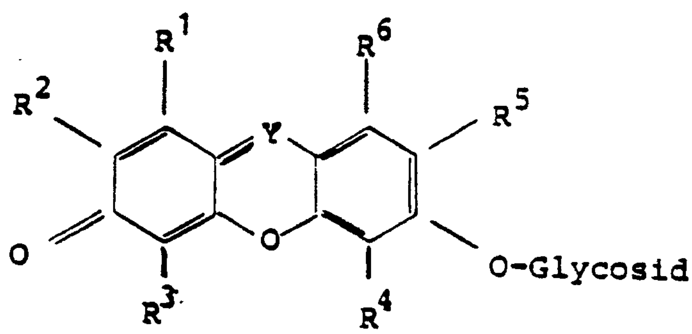

(I b)

in denen
$R^1$ Wasserstoff,
$R^2$, $R^3$ und $R^5$, die gleich oder verschieden sein können, Wasserstoff, Halogen oder eine $C_1$-$C_5$-Alkylgruppe,
$R^4$ und $R^6$, die gleich oder verschieden sein können, Wasserstoff, Halogen, eine Cyano-, $C_1$-$C_5$- Alkyl-, $C_1$-$C_5$- Alkoxy-, Carboxy-, $C_1$-$C_5$- Alkoxycarbonyl-, Carboxy-$C_1$-$C_5$- alkyl-, $C_1$-$C_5$- Alkoxycarbonyl- $C_1$-$C_5$-Alkyl- oder eine gegebenenfalls ein- oder zweifach substituierte Carboxamidogruppe oder die Gruppe

$-COO-(CH_2CH_2O)_n-R^7$

in der $R^7$ Wasserstoff oder eine $C_1$-$C_5$- Alkyl-Gruppe
und n eine ganze Zahl von 1 bis 4 bedeuten,
wobei $R^6$ zusätzlich eine Sulfonyl- oder Nitrogruppe vorstellen kann, und

Y Stickstoff oder die Gruppe N→O

bedeuten,

dadurch gekennzeichnet, daß man in an sich bekannter Weise Verbindungen der allgemeinen tautomeren Formel IIa und IIb

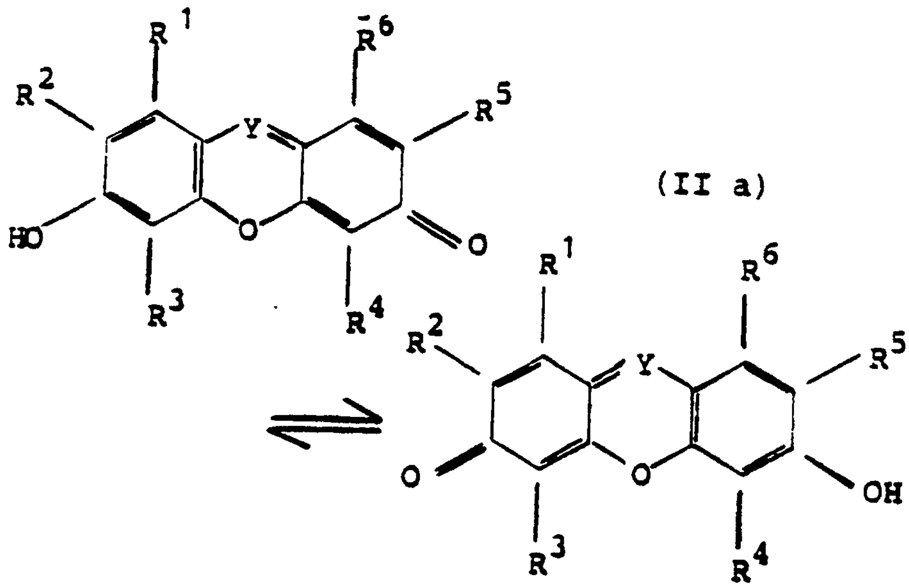

(II a)

(II b)

in denen

$R^1$ bis $R^6$ und Y die oben angegebene Bedeutung haben,

mit einem Mono- oder Oligosaccharid oder einem 1-Halogeno-Derivat hiervon, wobei jeweils alle Hydroxygruppen mit einer in der Kohlenhydratchemie üblichen Schutzgruppe substituiert sind, zu per-O-substituierten Glycosiden umgesetzt, aus denen durch Abspaltung der Schutzgruppen in an sich bekannter Weise die Glycoside von Resorufin-Derivaten der allgemeinen Formeln Ia bzw. Ib erhalten werden.

**5.** Verwendung der Glycoside von Resorufin-Derivaten gemäß einem der Ansprüche 1 oder 2 zur Bestimmung der Aktivität von entsprechenden Glycosidasen.

**6.** Verwendung der Glycoside von Resorufin-Derivaten gemäß einem der Ansprüche 1 oder 3 zur Bestimmung der Aktivität von Saccharidketten spaltenden Enzymen.

**7.** Diagnostisches Mittel zum Nachweis von Glycosidasen enthaltend ein oder mehrere chromogene oder/und fluorogene Substrate, eine geeignete Puffersubstanz sowie gegebenenfalls weitere üblicherweise verwendete Hilfsstoffe, dadurch gekennzeichnet, daß als chromogene und fluorogene Substrate Glycoside von Resorufin-Derivaten gemäß einem der Ansprüche 1 oder 2 eingesetzt werden.

**8.** Diagnostisches Mittel zum Nachweis von Saccharidketten spaltenden Enzymen, enthaltend ein oder mehrere chromogene oder/und fluorogene Stubstrate, eine geeignete Puffersubstanz, gegebenenfalls weitere üblicherweise verwendete Hilfsstoffe, eine geeignete Glycosidase sowie gegebenenfalls weitere Hilfsenzyme, dadurch gekennzeichnet, daß als chromogene und fluorogene Substrate Glycoside von Resorufin-Derivaten gemäß einem der Ansprüche 1 oder 3 eingesetzt werden.

**9.** Diagnostisches Mittel gemäß einem der Ansprüche 7 oder 8, dadurch gekennzeichnet, daß als zusätzliche Hilfsmittel Netzmittel, galenische Zusatzstoffe oder/und Gerüstbildner verwendet werden.

**10.** Resorufin-Derivate der allgemeinen tautomeren Formeln II'a und II'b

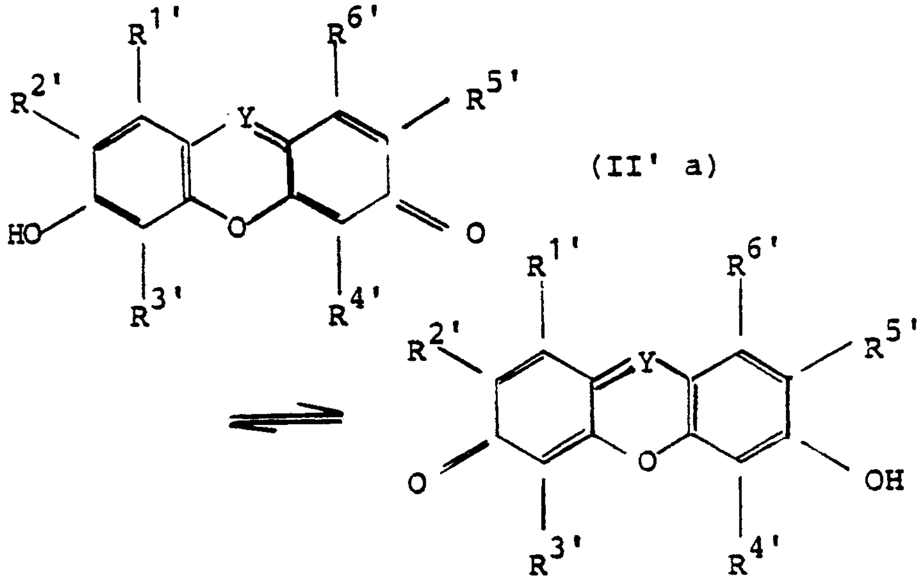

(II' a)

(II' b)

in denen
$R^{1'}$ Wasserstoff,
$R^{2'}$, $R^{3'}$ und $R^{5'}$, die gleich oder verschieden sein können, Wasserstoff, Halogen oder eine Niederalkylgruppe,
$R^{4'}$ und $R^{6'}$, die gleich oder verschieden sein können, Wasserstoff, Halogen, eine Cyano-, Niederalkyl-, Niederalkoxy-, Carboxy-, Niederalkoxycarbonyl-, Carboxyniederalkyl-, Niederalkoxycarbonyl-niederalkyl- oder eine gegebenenfalls ein- oder zweifach substituierte Carboxamidogruppe oder die Gruppe

$-COO-(CH_2CH_2O)_n-R^7$

in der $R^7$ Wasserstoff oder eine Niederalkyl-Gruppe
und n eine ganze Zahl von 1 bis 4 bedeuten, wobei
$R^{1'}$ bis $R^{6'}$ nicht alle gleichzeitig Wasserstoff bedeuten können, und

Y Stickstoff oder die Gruppe N→O,

bedeuten,
wobei mindestens einer der Reste $R^{4'}$ und $R^{6'}$ eine $C_1$-$C_5$-alkoxy, eine gegebenenfalls ein- oder zweifach substituierte Carboxamidogruppe oder die Gruppe $-COO-(CH_2CH_2O)_n-R^7$ bedeutet.

11. Verwendung von Resorufin-Derivaten gemäß Anspruch 1o zur Herstellung von Verbindungen gemäß einem der Ansprüche 1 oder 2.

## Claims

1. Glycosides of resorufin derivatives of the general formulae Ia or Ib

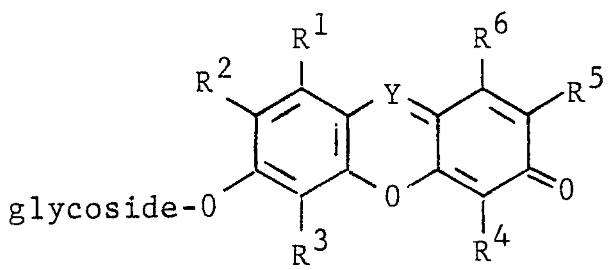

(Ia)

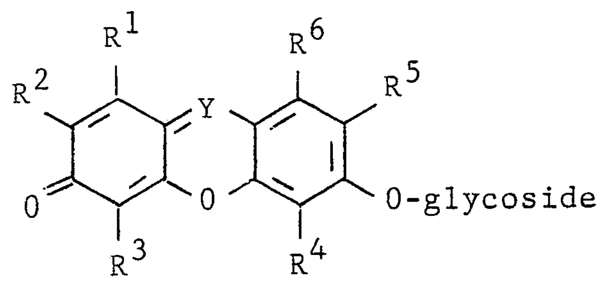

(Ib)

in which $R^1$ signifies hydrogen, $R^2$, $R^3$ and $R^5$, which can be the same or different, hydrogen, halogen or a $C_1$-$C_5$-alkyl group, $R^4$ and $R^6$, which can be the same or different, hydrogen, halogen, a cyano, $C_1$-$C_5$-alkyl, $C_1$-$C_5$-alkoxy, carboxyl, $C_1$-$C_5$-alkoxycarbonyl, carboxy-$C_1$-$C_5$-alkyl, $C_1$-$C_5$-alkoxy carbonyl-$C_1$-$C_5$-alkyl or a carboxamido group possibly substituted once or twice or the group $-COO-(CH_2CH_2O)_n-R^7$, in which $R^7$ signifies hydrogen or a $C_1$-$C_5$-alkyl group and n a whole number from 1 to 4, whereby $R^6$ can additionally represent a sulphonyl or nitro group and Y nitrogen or the group N→O.

**2.** Glycosides according to claim 1, characterised in that the glycoside residue represents a β-D-galactopyranoside, α-D-galactopyranoside, β-D-glucopyranoside, α-D-glucopyranoside or α-D-mannopyranoside residue.

**3.** Glycosides according to claim 1, characterised in that the glycoside residue represents an oligosaccharide with 2 to 10 monosaccharide units.

**4.** Process for the preparation of glycosides of resorufin derivatives of the general formulae Ia or Ib

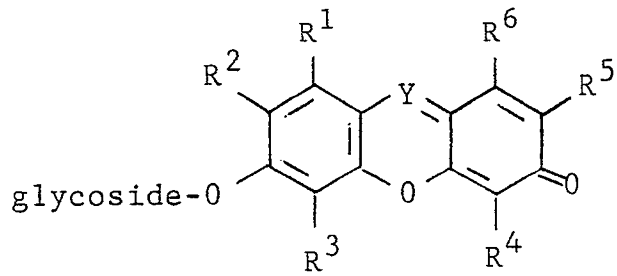

(Ia)

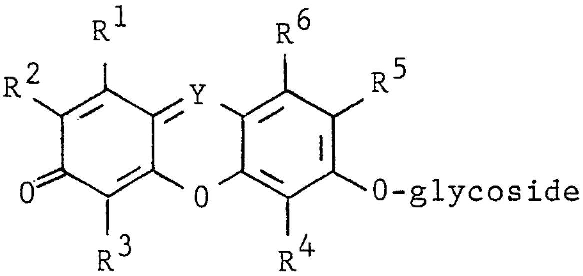

(Ib)

in which $R^1$ signifies hydrogen, $R^2$, $R^3$ and $R^5$, which can be the same or different, hydrogen, halogen or a $C_1$-$C_5$-alkyl group, $R^4$ and $R^6$, which can be the same or different, hydrogen, halogen, a cyano, $C_1$-$C_5$-alkyl, $C_1$-$C_5$-alkoxy, carboxyl, $C_1$-$C_5$-alkoxycarbonyl, carboxy-$C_1$-$C_5$-alkyl, $C_1$-$C_5$-alkoxycarbonyl-$C_1$-$C_5$-alkyl or a carboxamido group possibly substituted once or twice or the group $-COO-(CH_2CH_2O)_n-R^7$, in which $R^7$ signifies hydrogen or a $C_1$-$C_5$-alkyl group and n a whole number from 1 to 4, whereby $R^6$ can additionally represent a sulphonyl or nitro group and Y nitrogen or the group $N \rightarrow O$, characterised in that, in per se known manner, one reacts compounds of the general tautomeric formulae IIa or IIb

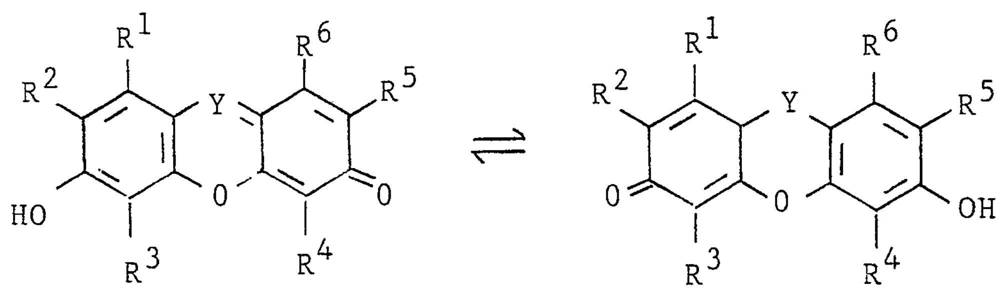

(IIa) (IIb)

in which $R^1$ to $R^6$ and Y have the above-given meaning, with a mono- or oligosaccharide or a 1-halo derivative hereof, whereby, in each case, all hydroxyl groups are substituted with a protective group usual in carbohydrate chemistry, to give per-O-substituted glycoside from which, by splitting off of the protective groups in per se known manner, glycosides of resorufin derivatives of the general formula Ia or Ib are obtained in per se known manner.

5. Use of the glycosides of resorufin derivatives according to one of claims 1 or 2, for the determination of the activity of corresponding glycosidases.

6. Use of the glycosides of resorufin derivatives according to one of claims 1 or 3 for the determination of the activity of enzymes cleaving saccharide chains.

7. Diagnostic agent for the detection of glycosidases containing one or more chromogenic and/or fluorogenic substrates, a suitable buffer substance, as well as possibly further usually employed

adjuvants, characterised in that, as chromogenic and fluorogenic substrates, glycosides of resorufin derivatives according to one of claims 1 or 2 are used.

**8.** Diagnostic agent for the detection of enzymes cleaving saccharide chains, containing one or more chromogenic and/or fluorogenic substrates, a suitable buffer substance, possibly further usually employed adjuvants, a suitable glycosidase, as well as possibly further adjuvant enzymes, characterised in that, as chromogenic and fluorogenic substrates, glycosides of resorufin derivatives according to one of claims 1 or 3 are used.

**9.** Diagnostic agent according to one of claims 7 or 9, characterised in that wetting agents, galenical additives and/or structure formers are used as additional adjuvants.

**10.** Resorufin derivatives of the general tautomeric formulae II'a and II'b

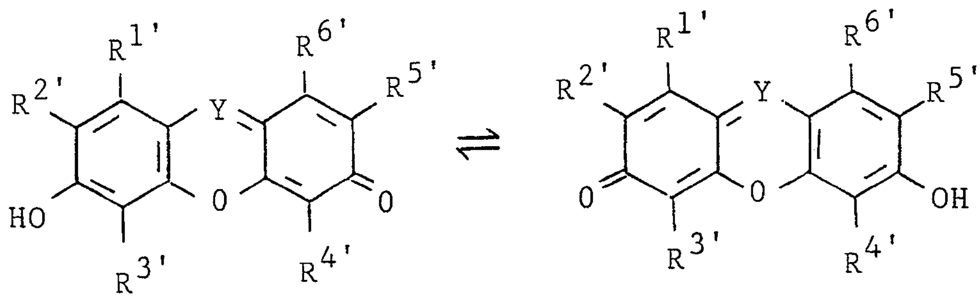

(II'a) (II'b)

in which $R^{1'}$ signifies hydrogen, $R^{2'}$, $R^{3'}$ and $R^{5'}$, which can be the same or different, hydrogen, halogen or a lower alkyl group, $R^{4'}$ and $R^{6'}$, which can be the same or different, hydrogen, halogen, a cyano, lower alkyl, lower alkoxy, carboxyl, lower alkoxycarbonyl, carboxy lower alkyl, lower alkoxy carbonyl lower alkyl or a carboxamido group possibly substituted once or twice or the group $-COO-(CH_2CH_2O)_n-R^7$, in which $R^7$ signifies hydrogen or a lower alkyl group and n a whole number from 1 to 4, whereby $R^{1'}$ to $R^{6'}$ cannot all simultaneously signify hydrogen, and Y nitrogen or the group N → O, whereby at least one of the radicals $R^{4'}$ and $R^{6'}$ signifies a $C_1$-$C_5$-alkoxy, a carboxamido group possibly substituted once or twice or the group $-COO-(CH_2CH_2O)_n-R^7$.

**11.** Use of resorufin derivatives according to claim 10 for the preparation of compounds according to one of claims 1 or 2.

## Revendications

**1.** Glycosides de dérivés de résorufine de formule générale Ia ou Ib

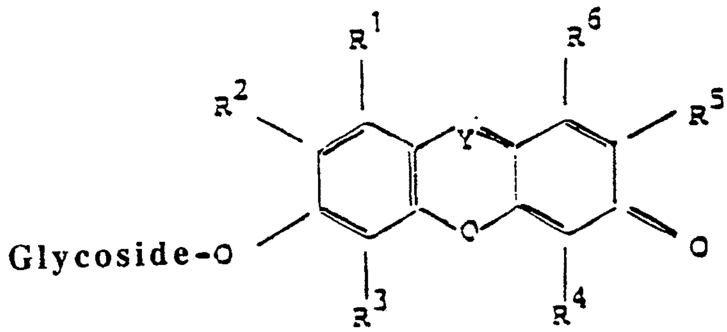

(I a)

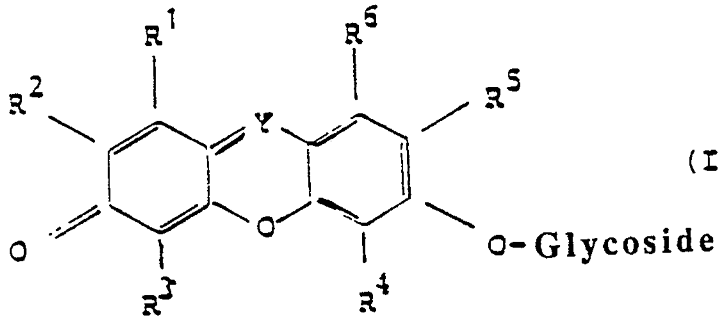

(I b)

dans laquelle

$R^1$ représente l'hydrogène,
$R^2$, $R^3$ et $R^5$, qui peuvent être identiques ou différents, représentent l'hydrogène, un halogène ou un groupe alkyle en $C_1$-$C_5$,
$R^4$ et $R^6$, qui peuvent être identiques ou différents, représentent l'hydrogène, un halogène, un groupe cyano, alkyle en $C_1$-$C_5$, alcoxy en $C_1$-$C_5$, carboxy, alcoxy($C_1$-$C_5$)carbonyle, carboxyalkyle($C_1$-$C_5$), alcoxy($C_1$-$C_5$)carbonylalkyle($C_1$-$C_5$) ou un groupe carboxamido éventuellement substitué une ou deux fois, ou le groupe

$-COO-(CH_2CH_2O)_n-R^7$

dans lequel $R^7$ représente l'hydrogène ou un groupe alkyle en $C_1$-$C_5$
et n vaut un nombre entier de 1 à 4,
$R^6$ pouvant représenter en outre un groupe sulfonyle ou nitro, et
Y représente l'azote ou le groupe $N \rightarrow O$.

2. Glycosides selon la revendication 1, caractérisés en ce que le reste glycoside représente un reste $\beta$-D-galactopyranoside, $\alpha$-D-galactopyranoside, $\beta$-D-glucopyranoside, $\alpha$-D-glucopyranoside ou $\alpha$-D-mannopyranoside.

3. Glycosides selon la revendication 1, caractérisés en ce que le reste glycoside représente un oligosaccharide comportant 2 à 10 motifs monosaccharides.

4. Procédé pour la préparation de glycosides de dérivés de résorufine de formule générale Ia ou Ib,

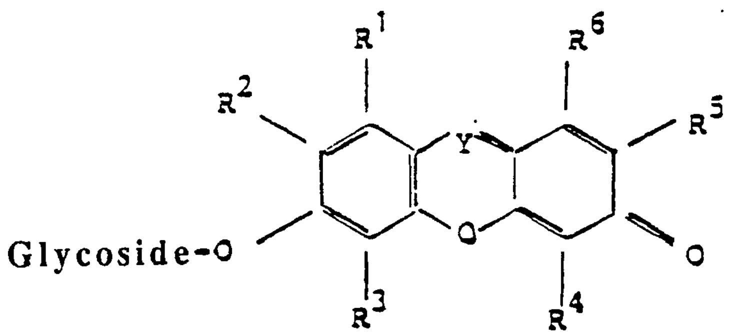

(I a)

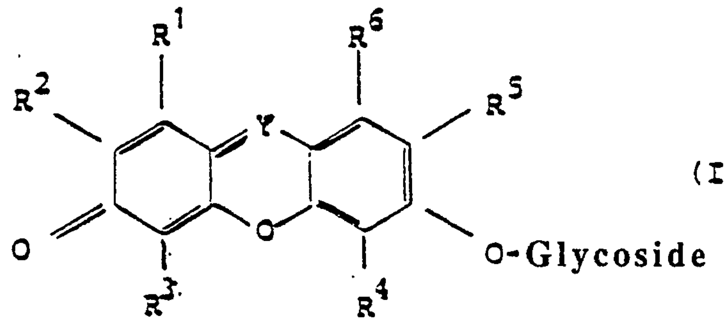

(I b)

dans laquelle

$R^1$ représente l'hydrogène,
$R^2$, $R^3$ et $R^5$, qui peuvent être identiques ou différents, représentent l'hydrogène, un halogène ou un groupe alkyle en $C_1$-$C_5$,
$R^4$ et $R^6$, qui peuvent être identiques ou différents, représentent l'hydrogène, un halogène, un groupe cyano, alkyle en $C_1$-$C_5$, alcoxy en $C_1$-$C_5$, carboxy, alcoxy($C_1$-$C_5$)carbonyle, carboxyalkyle($C_1$-$C_5$), alcoxy($C_1$-$C_5$)carbonylalkyle($C_1$-$C_5$) ou un groupe carboxamido éventuellement substitué une ou deux fois, ou le groupe

$-COO-(CH_2CH_2O)_n-R^7$

dans lequel $R^7$ représente l'hydrogène ou un groupe alkyle en $C_1$-$C_5$
et n vaut un nombre entier de 1 à 4,
$R^6$ pouvant représenter en outre un groupe sulfonyle ou nitro, et
Y représente l'azote ou le groupe N → O,
caractérisé en ce que de manière connue en soi, on fait réagir des composés de formules générales tautomères 2a et 2b

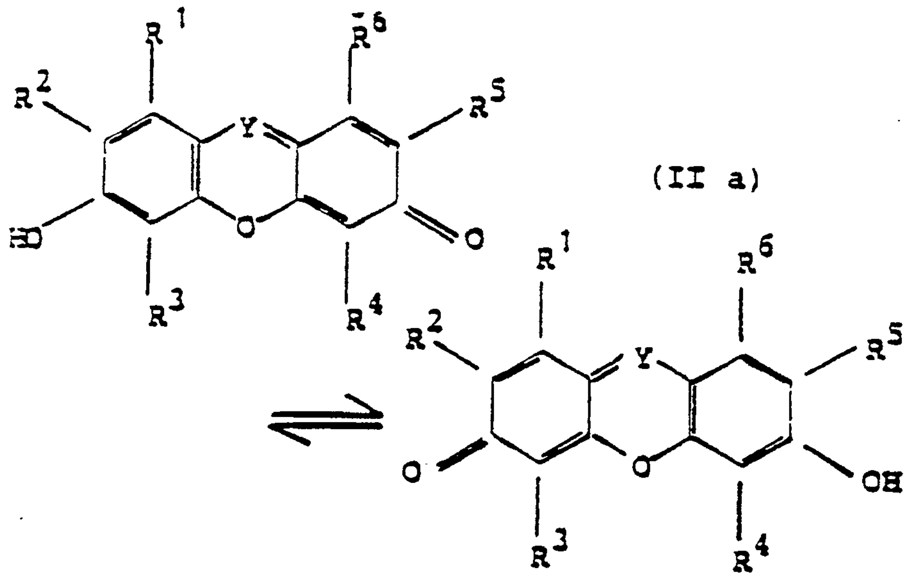

(II a)

(II b)

dans lesquelles

$R^1$ à $R^6$ et Y ont les significations mentionnées ci-dessus,

avec un mono- ou oligosaccharide ou avec un de leurs dérivés 1-halogéné, chacun des groupes hydroxy étant substitué avec un groupe protecteur usuel dans la chimie des hydrates de carbone, pour obtenir des glycosides per-O-substitués, à partir desquels, par séparation des groupes protecteurs de manière connue en soi, on obtient les glycosides de dérivés de résorufine, de formule générale Ia ou Ib.

**5.** Utilisation des glycosides de dérivés de résorufine selon l'une quelconque des revendications 1 ou 2, pour la détermination de l'activité des glycosidases correspondantes.

**6.** Utilisation des glycosides de dérivés de résorufine selon l'une quelconque des revendications 1 ou 3, pour la détermination de l'activité d'enzymes coupant les chaînes saccharidiques.

**7.** Agent de diagnostic pour la détermination de glycosidases contenant un ou plusieurs substrats chromogènes et/ou fluorogènes, une substance tampon appropriée ainsi qu'éventuellement d'autres adjuvants usuels, caractérisé en ce que comme substrat chromogène et fluorogène, on utilise des glycosides de dérivés de résorufine selon l'une des revendications 1 ou 2.

**8.** Agent de diagnostic pour la détermination d'enzymes coupant des chaînes saccharidiques, contenant un ou plusieurs substrats chromogènes et/ou fluorogènes, une substance tampon appropriée, éventuellement d'autres adjuvants usuels, une glycosidase appropriée ainsi qu'éventuellement d'autres enzymes auxiliaires, caractérisé en ce que comme substrat chromogène et fluorogène, on utilise des glycosides de dérivés de résorufine selon l'une des revendications 1 ou 3.

**9.** Agent de diagnostic selon l'une des revendications 7 ou 8, caractérisé en ce que comme adjuvant supplémentaire on utilise un agent mouillant, des adjuvants galéniques ou/et des agents de structure.

**10.** Dérivés de résorufine de formules générales tautomères II'a et II'b

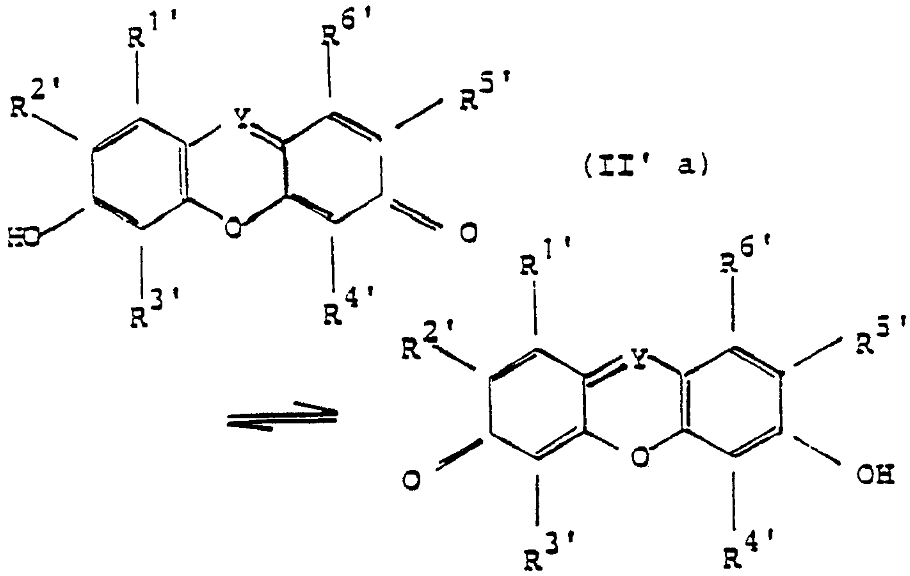

(II' a)

(II' b)

dans lesquelles

$R^{1'}$ représente l'hydrogène,
$R^{1'}$, $R^{3'}$ et $R^{5'}$, qui peuvent être identiques ou différents, représentent l'hydrogène, un halogène ou un groupe alkyle inférieur,
$R^{4'}$ et $R^{6'}$, qui peuvent être identiques ou différents, représentent l'hydrogène, un halogène, un groupe cyano, un groupe alkyle inférieur, alcoxy inférieur, carboxy, alcoxycarbonyle inférieur, carboxyalkyle inférieur, alcoxycarbonyle inférieur-alkyle inférieur ou un groupe carboxamido éventuellement substitué une ou deux fois ou le groupe

$-COO-(CH_2CH_2O)_n-R^7$

dans lequel $R^7$ représente l'hydrogène ou un groupe alkyle inférieur et n vaut un nombre entier de 1 à 4, $R^{1'}$ à $R^{6'}$ ne pouvant représenter tous à la fois l'hydrogène, et
Y représente l'azote ou le groupe N → O,
étant entendu qu'au moins un des groupes $R^{4'}$ et $R^{6'}$ représente un groupe alcoxy en $C_1$-$C_5$, un groupe carboxamido éventuellement substitué une ou deux fois ou le groupe

$-COO-(CH_2CH_2O)_n-R^7$.

**11.** Utilisation des dérivés de résorufine selon la revendication 10, pour la préparation de composés selon l'une des revendications 1 ou 2.